(19) **Europäisches Patentamt / European Patent Office / Office européen des brevets**

(11) **EP 4 728 881 A1**

(12) **EUROPEAN PATENT APPLICATION**
published in accordance with Art. 153(4) EPC

(43) Date of publication:
**22.04.2026 Bulletin 2026/17**

(21) Application number: **24822768.8**

(22) Date of filing: **14.06.2024**

(51) International Patent Classification (IPC):
**A23L 33/125** (2016.01)   **A61K 36/068** (2006.01)
**A61P 39/06** (2006.01)

(86) International application number:
**PCT/CN2024/099118**

(87) International publication number:
**WO 2024/255820 (19.12.2024 Gazette 2024/51)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **15.06.2023   CN 202310716796**

(71) Applicants:
- **Hangzhou Zhongmeihuadong Pharmaceutical Jiangdong Co., Ltd**
  **Hangzhou, Zhejiang 311500 (CN)**
- **Hangzhou Zhongmeihuadong Pharmaceutical Co., Ltd.**
  **GongShu District**
  **HangZhou, Zhejiang 310011 (CN)**

(72) Inventors:
- **XU, Feng**
  **Hangzhou Hangzhou, Zhejiang 311500 (CN)**

- **TENG, Yi**
  **Hangzhou Hangzhou, Zhejiang 311500 (CN)**
- **WANG, Hongyan**
  **Hangzhou Hangzhou, Zhejiang 311500 (CN)**
- **ZHANG, Hui**
  **Hangzhou Hangzhou, Zhejiang 311500 (CN)**
- **ZHANG, Xuan**
  **Hangzhou Hangzhou, Zhejiang 311500 (CN)**
- **CHEN, Zhijie**
  **Hangzhou Hangzhou, Zhejiang 311500 (CN)**
- **LI, Yapei**
  **Hangzhou Hangzhou, Zhejiang 311500 (CN)**
- **WANG, Jie**
  **Hangzhou Hangzhou, Zhejiang 311500 (CN)**
- **JIANG, Hui**
  **Hangzhou Hangzhou, Zhejiang 311500 (CN)**

(74) Representative: **Maiwald GmbH**
**Elisenhof**
**Elisenstraße 3**
**80335 München (DE)**

(54) **CORDYCEPS SINENSIS FERMENTATION COMPOSITION**

(57)    Disclosed is a Cordyceps sinensis fermentation composition, comprising erythritol having a content of more than 1 wt%, preferably, 1-6 wt%, preferably, 2-5 wt%, further preferably, 2.5-4 wt%. The Cordyceps sinensis fermentation composition has an excellent antioxidant effect and a synergistic technical effect.

FIG. 1

EP 4 728 881 A1

**Description**

The present disclosure claims priority to the Chinese patent application with an application number 202310716796X and titled "Cordyceps sinensis Fermentation Composition", filed with the China National Intellectual Property Administration on June 15, 2023, the disclosures of which are hereby incorporated by reference.

**Technical Field**

The present disclosure relates to a Cordyceps sinensis fermentation composition, and belongs to the technical field of microbial fermentation.

**Background**

Erythritol is a four-carbon polyol with its chemical name being 1,2,3,4-butanetetraol, molecular formula being $C_4H_{10}O_4$, and relative molecular mass being 122.12. As a natural sweetener, erythritol exists widely in nature. It occurs naturally in small amounts in fruits and vegetables such as pears, grapes, and mushrooms, as well as in fermented foods such as wine, beer, soy sauce, and sake. Additionally, it is present in small quantities in animal serum and eyeballs.

The erythritol features a low molecular weight and strong hydrophilicity. It does not contain a reductive aldehyde group and thus exhibits high stability to heat and acid. It is readily soluble in water, with a solubility being 37% at 25°C. Upon dissolving in the water, it absorbs heat, producing a cooling sensation when a human eats it in a solid form. With good crystallinity and low hygroscopicity, the erythritol remains non-hygroscopic even at 20°C and 90% relative humidity. Due to its sweetness being 60-80% that of sucrose, the erythritol has a clean and pure sweet taste, and is free from undesirable bitterness.

A low caloric value and a unique metabolic pathway are among metabolic characteristics of the erythritol. The erythritol has an energy coefficient of 0.88 kJ/g, making it one of the lowest-calorie polyol sweeteners currently in use. After the erythritol is ingested by a human body, approximately 80-90% of it is absorbed in small intestine via passive diffusion, while the remaining 10% enters large intestine and is fermented as a carbon source. Moreover, as an intake dose of the erythritol increases, a proportion of the erythritol entering the large intestine also increases. The erythritol absorbed by the small intestine of the human body is widely distributed throughout the body's tissues. Approximately 5-10% of the absorbed erythritol is oxidized into erythrose in blood, followed by rapid further oxidization for producing the erythrulose, which is finally excreted in urine. The remaining unoxidized erythritol cannot be decomposed by an enzyme system in the body and thus can only be filtered from the blood through kidney and then excreted in the urine. The erythritol entering the large intestine is fermented by intestinal floras into short-chain fatty acids (SCFAs). After the SCFAs are absorbed into an intestinal tract, they can improve a diet-induced metabolic disorder through their anti-inflammatory efficacy, offering a protecting effect on obesity and inflammatory bowel disease.

Good intestinal tolerance is another characteristic of the erythritol. Compared with other sugar alcohols, the erythrose's maximum non-laxative dose ranges from 0.66 g/kgbw to 1.0+ g/kgbw, which is significantly higher than those of other sugar alcohols. Studies on human intestinal tolerance have shown that healthy volunteers aged 18-24 years exhibited good intestinal tolerance without any adverse symptom if they consumed 20-35 g of the erythritol in a liquid form; and when an intake reached 50 g, symptoms such as diarrhea and nausea were observed. Meanwhile, clinical studies have shown that the diarrhea and/or a severe intestinal symptom is not observed in children aged 4-6 years who consumed beverages containing 15 g of the erythritol. In addition, it is to be noted that the diarrhea caused by excessive intake of the sugar alcohols is not a disease, but rather a simple osmotic response to slowly absorbed carbohydrates in an intestinal lumen. That is, sugar alcohols unabsorbed in the small intestine may cause bloating, the diarrhea, and flatulence due to an osmotic effect. Some people may experience these symptoms after one consumption. With continued consumption, most people develop a certain degree of tolerance to the sugar alcohols, and related gastrointestinal symptoms tend to diminish or disappear. In addition, the erythritol also has positive significance for oral health protection. Studies have shown that the erythritol is not only unutilized by Streptococcus mutans in an oral cavity, but it can also inhibit growth and adhesion of Streptococcus mutans in the oral cavity, thereby exerting beneficial effects on the oral cavity. In a three-year clinical trial, subjects treated with the erythritol have showed significantly lower levels of acetic acid and propionic acid in dental plaques, as well as a markedly reduced count of plaque Streptococcus mutans, compared with subjects treated with xylitol or sorbitol.

Currently, the erythritol is mainly produced industrially through microbial fermentation. Many microorganisms in nature are capable of synthesizing the erythritol, including osmophilic yeasts such as Moniliella pollinis, Trigonopsis, Trichosporon, and Yarrowia lipolytica, as well as bacteria such as Oenococcus oeni, Leuconostoc oenos, and Lactobacillus. Synthetic pathways of the erythritol differ between the yeasts and the bacteria. In the yeast, the erythritol is synthesized via a pentose phosphate pathway, where erythrose-4-phosphate is dephosphorylated to form the erythrose, which is then reduced to the erythritol by the action of the erythrose reductase. In one bacterium, the erythritol can be produced through a reduced coenzyme II (NADPH) regeneration step in a phosphoketolase pathway.

Mannitol, also known as D-mannitol, hexanehexol, or manna sugar, is an isomer of sorbitol. The mannitol is present in both natural Cordyceps sinensis and fermented Cordyceps sinensis bacterial powder, differing in content. The mannitol, as a typical hexavalent sugar alcohol, absorbs heat upon dissolution. After it is digested and absorbed in the human body, the mannitol first reacts with $NAD^+$ to produce acetyl-CoA and fructose. Through the action of fructokinase, two forms of phosphofructose are produced, and enter a triose phosphate cycle to complete their physiological metabolism of the mannitol. It can be seen that a metabolic process of the mannitol is quite special, as it does not require participation of insulin throughout the process; consequently, it does not cause a significant fluctuation in blood glucose concentration. Therefore, the mannitol can be regarded as a low-calorie food additive.

The mannitol is also a low-calorie and low-sugar sweetener with a cool taste. Its sweetness is about 60% that of the sucrose and 76% that of the xylitol. Due to its unique metabolic pathway, it can also be taken by patients with diabetes and obesity. It can serve as both the sweetener and a flavoring agent in chewing gum. As a sweetener and an additive for sugar-free foods, it is used in production and processing of various foods, helps preserve fruit flavors, and serves as an aromatizer of dried fruits.

The mannitol can be used in foods and daily chemical products for preventing tooth decay or improving dental caries, because, as the sweetener, it does not provide nutrients for the microorganisms in the oral cavity and can further inhibit growth and reproduction of Streptococci.

The mannitol is also a potent free radical scavenger. It can rapidly eliminate a portion of hydroxyl radicals in a free radical chain reaction. When acting together, the mannitol and these radicals form mannitol free radicals with low toxicity, which are subsequently detoxified through disproportionation. This prevents an irreversible damage on tissues in a penumbra region, thereby alleviating a damage on neurological function; and the mannitol can be used clinically as a neuroprotective agent. In addition, the mannitol inhibits production of oxygen free radicals. It can effectively scavenge free radicals produced under hypoxic conditions in brain tissues, myocardial cells, and limb muscle tissues. Furthermore, it can reduce the blood concentration, decrease erythrocyte aggregation and cerebral vascular resistance, improve microcirculation, and enhance perfusion of organ tissues.

Ergothioneine (EGT or ERG) is a naturally rare chiral amino acid-type strong antioxidant first discovered in 1909 in fungus Claviceps purpurea. It is a water-soluble and naturally occurring thiol compound and has been recognized as an endogenous antioxidant and a protective factor that helps cells resist oxidative stress. The ergothioneine possesses multiple functions, including an antioxidant function, a free radical scavenging function, metal ion chelation, protection against ultraviolet radiation damage, regulation of an intracellular redox reaction, participation in cellular energy regulation, and use as a physiological cell protectancellular physiological protector. It is therefore an important active substance in vivo.

The ergothioneine is distributed in certain tissues and organs of mammals, primarily found in red blood cells and semen of some animals. Its content in human and mammalian tissues ranges from 1 mM to 2 mM (equivalent to 229.3-458.6 mg/L) (Melville, 1959; Hartman, 1990, or The Natural Antioxidant Ergothioneine, Lipid Oxidation, 2013). This indicates that the ergothioneine may function in vivo as a non-toxic antioxidant. A deficiency of the ergothioneine in the cells renders them highly susceptible to oxidative stress, which can lead to increased mitochondrial DNA damage, protein oxidation, and lipid peroxidation. However, to date, no experimental study has demonstrated that any animal species can synthesize this substance; and there certainly are pathways within the animal cells for uptake and retention of the ergothioneine. With its unique biological and pharmacological properties, as well as advantages of safety, stability, etc., the ergothioneine shows promising application prospects in the fields of cosmetics, functional foods, pharmaceuticals, and biomedicine, and has attracted widespread attention from researchers both domestically and internationally.

The ergothioneine is found primarily in the mushrooms. Melville D. B. and Eieh S. have found through studies that there was also the ergothioneine in cereal plants. Subsequent studies by Melville D. B., Genghof D. S., et al. have shown that the ergothioneine is a common component of many microbial cells, can be synthesized in several fungi, but cannot be synthesized in the bacteria. Because the mushrooms are a rich source of antioxidants including the ergothioneine, this provides an additional reason for incorporating the mushrooms into human diets. For example, Shin-Yu Chen et al. (Contents of lovastatin, γ-aminobutyric acid and ergothioneine in mushroom fruiting bodies and mycelia, Food Science and Technology, 2012, 47: 274-278) have reported that among mushroom fruiting bodies, Pleurotus citrinopileatus, P. ostreatus (Korea), P. ostreatus (Taiwan, China) and Pleurotus salteostramineus have the highest ergothioneine contents (at 2850.7 mg/kg, 1829.4 mg/kg, 1458.4 mg/kg, and 1245.0 mg/kg respectively). In contrast, among mycelia, the highest content of the ergothioneine is found in Pleurotus eryngii at 1514.6 mg/kg. To further increase the ergothioneine content in the mushrooms, Wi Yong Lee et al. (Ergothioneine Contents in Fruiting Bodies and Their Enhancement in Mycelial Cultures by the Addition of Methionine, Mycobiology, 2009, 37(1): 43-47) have reported that adding methionine to mycelial culture enhances the ergothioneine content in the mushroom fruiting bodies. A difference in the ergothioneine content between the mushroom species reaches up to 92.3-fold. Adding 2 mM of the methionine (Met) to a mycelial culture medium can increase the ergothioneine content in the tested mushroom species, indicating that the methionine is a beneficial additive for promoting ergothioneine production in the mushrooms.

Cordyceps militaris is also known as North Cordyceps sinensis, and Scientific name of Cordyceps militaris is Cordyceps militaris (Vuill.) Fr., the english name is pupa insect grass. Its strain of anamorph is Verticillium, while Liang Zongqi (1990) considers it to belong to Cephalosporium. Patent WO2014/055035 discloses a Cordyceps militaris strain CBS 132098, as well as a fruiting body with biological activity, a mycelial biomass, and an extract thereof. The ergothioneine content in the mycelial biomass of Cordyceps militaris is 130.65 mg/kg (0.013%), corresponding to fourth-level classification from Chen et al. (Chang et al., 2012). Bai-Xiong et al. (Enhancement of ergothioneine production by discovering and regulating its metabolic pathway in Cordyceps militaris, Microb Cell Fact, 2022 Aug 23; 21(1):169) constructs a novel ergothioneine synthetic pathway. By further introducing this pathway into a genome of Cordyceps militaris, a component yield of an engineered strain is successfully increased, achieving a maximum ergothioneine content of up to 2.5 g/kg dry weight.

Cordyceps sinensis (Cordyceps sinensis(Borkeler)sacc.) and Cordyceps militaris are two distinct species. Cordyceps sinensis exhibits a high host specificity: it can parasitize only larvae of Hepialus armoricanus Oberthur. Cordyceps sinensis has a scientific name of Cordyceps sinensis (Berk) Sacc., and an English name of Chinese caterpillar fungus. Its anamorphic name has been confirmed by experts and scholars as Hirsutella sinensis. Hui-Chen Lo et al. (A Systematic Review of the Mysterious Caterpillar Fungus Ophiocordyceps sinensis in DongChongXiaCao and Related Bioeffective components, Journal of Traditional and Complementary Medicine 2013(3)1:16-32) believe that a fruiting body of wild Cordyceps sinensis contains detectable ergothioneine, which is considered a secondary metabolite produced in fungal growth rather than a small molecular organic compound essential for normal growth and development of fungi. Although Shin-Yu Chen et al. have reported that a Cordyceps sinensismycelium contains 142.0 $\pm$ 38.5 IJK ergothioneine, Shin-Yi Lin et al. (Comparative Study of Contents of Several Bioactive Components in Fruiting Bodies and Mycelia of Culinary-Medicinal Mushrooms, International Journal of Medicinal Mushrooms, 2013(15)3: 315-323) have reported that the ergothioneine content in an Ophiocordyceps sinensis mycelium is only 35.70$\pm$1.28 K, and that in the fruiting body is even lower, at only 12.20$\pm$1.51 E. Nachshol Cohen et al. (Chemical Composition and Nutritional and Medicinal Value of Fruit Bodies and Submerged Cultured Mycelia of Culinary-Medicinal Higher Basidiomycetes Mushrooms, International Journal of Medicinal Mushrooms, 2014(16)3: 273-291) have reported that the ergothioneine content in the Ophiocordyceps sinensis mycelium is 52.18$\pm$2.75 $\mu$g/g. Consequently, to date, there is no wild or natural Ophiocordyceps sinensis that stably contains a high content of the ergothioneine, and also no wild or natural Ophiocordyceps sinensis mycelium that stably contains a high content of the ergothioneine. Furthermore, Ophiocordyceps sinensis belongs to the phylum Ascomycota, class Sordariomycetes, order Hypocreales, family Ophiocordycipitaceae, and genus Ophiocordyceps (Technical Specifications for Pollution-Free Cultivation and Production of Chinese Medicinal Materials, 2018). Natural Ophiocordyceps sinensis is scarce in source and high in price, so using the Ophiocordyceps sinensis mycelium as a substitute for the natural Ophiocordyceps sinensis represents a technological trend. For decades, researchers have isolated fungi from Ophiocordyceps sinensis collected from various regions, involving over 30 species across 10 genera, such as Paecilomyces hepiali, Paecilomyces sinensis, Mortierella hepiali, Cephalosporium sinensis, Hirsutella sinensis, Tolypocladium sinense, and Chrysosporium sinense. Studies on the metabolome of Ophiocordyceps sinensis at different growth stages (Zhang Zhou, Anhui Agricultural University, 2018) have indicated significant differences in metabolites between different parts of Ophiocordyceps sinensis, as well as significant differences in one part across different developmental stages. These differences are more pronounced in an early stage of development. Therefore, fermentation of Ophiocordyceps sinensis and a combination of beneficial components remain technical problems that need to be solved.

**Summary**

The inventor has unexpectedly found that, through process optimization, a provided Cordyceps sinensis fermentation composition includes more than 1% (weight percentage) of erythritol, preferably 1-6% (weight percentage), further preferably 2-5% (weight percentage), more further preferably 2.5-4%. Furthermore, it is commonly believed by those skilled in the art that the erythritol serves merely as a sweetener. However, the present disclosure, through studies, has unexpectedly found that combination of the erythritol and adenosine jointly exhibits a synergistic effect on hydroxyl radical scavenging rate, thereby enhancing an overall effect of the Cordyceps sinensis fermentation composition. Particularly in a case of the erythritol's content falling within a range specified in the present disclosure, its combination with the adenosine exhibits an outstanding synergistic effect on the hydroxyl radical scavenging rate.

In one aspect, the present disclosure obtains Cordyceps sinensis producing the erythritol by screening Cordyceps sinensis, and then obtains a fermentation composition including the erythritol through fermentation. In another aspect, the present disclosure enables stable and sufficient presence of the erythritol in Cordyceps sinensis mycelium or bacterial powder by employing a low-temperature liquid fermentation technology. In yet another aspect, the present disclosure achieves reasonable presence of effective components such as the erythritol and the adenosine through a low-temperature fermentation process. This not only enhances stability of the Cordyceps sinensis fermentation composition, but also provides a new direction for utilizing fermented Cordyceps sinensis bacterial powder.

Specifically, in one aspect, for the present disclosure, the inventor has isolated Hirsutella sinensis capable of producing the erythritol from natural Cordyceps sinensis by screening strains of Cordyceps sinensis. In another aspect, the inventor has obtained a stable fermented mycelium or bacterial powder with a high content of stably existing erythritol through fermentation of Hirsutella sinensis. Through liquid fermentation culture, the present disclosure achieves, for the first time, erythritol at a content comparable to that found in natural Cordyceps sinensis, thereby enabling the Cordyceps sinensis fermentation composition to exert broader functionalities.

A Hirsutella sinensis strain is of an anamorphic generation of the fungus Cordyceps sinensis (Berk) Sacc., which belongs to the family Clavieps purpurea (Fr.)Tul., and is isolated from fresh Cordyceps sinensis collected on the Qinghai-Tibet Plateau. Hirsutella sinensis described in the present disclosure may be any strain of Hirsutella sinensis. For example, it may be purchased from a commercial platform or obtained through independent screening and isolation, provided that it can be identified as Hirsutella sinensis following principles such as microbial molecular genetics. Commercial sources include, but are not limited to, China Center for Type Culture Collection (CCTCC) or China General Microbiological Culture Collection Center (CGMCC) for obtaining Hirsutella sinensis. Examples from the China Center for Type Culture Collection (CCTCC) include, but are not limited to, Hirsutella sinensis CCTCC No: M 2011278. Examples from the China General Microbiological Culture Collection Center (CGMCC) include, but are not limited to, Hirsutella sinensis CGMCC 3.14240 and Hirsutella sinensis CGMCC 3.14243. For example, standard strains of Ophiocordyceps sinensis (Hirsutella hepiali or Hirsutella sinensis) or Cordyceps sinensis (Hirsutella hepiali or Hirsutella sinensis) purchased from the China Center of Industrial Culture Collection (CICC) include, but are not limited to, strains with numbers: CICC 14016, CICC 14017, CICC 14088, CICC 14089, CICC 14090, CICC 14094, CICC 14096, CICC 14091, CICC 14092, CICC 14093, CICC 14095, CICC 14097, CICC 14098, CICC 14099, CICC 14100, CICC 14101, CICC 14102, CICC 14103, CICC 14104, CICC 14105, CICC 14106, CICC 14107, CICC 14108, CICC 14109, CICC 14110, CICC 14111, CICC 14112, CICC 14113, CICC 14114, CICC 14115, CICC 14117, CICC 14118, CICC 14119, CICC 14120, and CICC 50002. Examples of Ophiocordyceps sinensis (Hirsutella hepiali or Hirsutella sinensis), Cordyceps sinensis, or Hirsutella sinensis purchased from Tesuobio include, but are not limited to, products with codes: TS324838, TS325049, TS325054, TS326189, TS326195, TS326196, TS326197, TS326198, TS326199, TS326200, TS326201, TS326202, TS326203, TS326204, TS326205, TS326206, TS326207, TS326210, TS326212, TS326213, TS326214, TS326408, TS326410, TS326412, TS342791, TS342792, TS342793, TS342794, TS342795, TS342796, TS342797, TS342798, TS342800, TS342809, TS361737, and TS392986.

Hirsutella sinensis has a growth cycle longer than that of the vast majority of fungi. It typically grows in environments at 14-20°C and is classified as a mesophilic to low-temperature fungus. If Hirsutella sinensis is cultured on a solid medium, its diameter can reach up to 1.5 cm, and its colony is of a rubbery texture, appearing flesh-colored or milky-white. In an early stage of growth, aerial mycelia are present on the colony's surface. Over time, the colony's surface develops a smooth furrowed appearance, with the aerial mycelia gradually diminishing. If Hirsutella sinensis is cultured in a liquid medium, its mycelia appear as filaments or grey pellets in varying sizes. As culture proceeds, a brown secretion is released into the medium. The mycelial pellets are of a tough and unloosened texture, and are elastic and hollow inside. It is known that the mycelia obtained from liquid fermentation culture of Hirsutella sinensis is dried, and then becomes fermented Cordyceps sinensis bacterial powder. In this way, various active substances can be obtained, including polysaccharides, amino acids, nucleosides, sterols, mannitol, trace metals, etc. Among them, marker components include the adenosine, the mannitol, the polysaccharides, the amino acids, etc. The fermented Cordyceps sinensisbacterial powder exhibits various effects, including enhancing immune function, exerting anti-fibrosis activity, protecting a renal function, resisting tumors and inflammation, etc., and also provides protective effects on organs such as kidney, lung, and liver.

Fermentation in the present disclosure refers to a process of culturing an existing strain of the anamorphic generation of Chinese Cordyceps sinensis, namely Hirsutella sinensis, with slant culture, seed flask/shake flask culture, and fermentation culture.

Slant culture of the present disclosure involves allowing Hirsutella sinensis to grow on a slant surface of a solid medium; and inoculating and spreading Hirsutella sinensis onto the slant surface for culture at a low temperature, for example, at 8-18°C for 20-60 d, preferably, at 10-16°C for 30-50 d, wherein a slant culture medium includes main components including one or more of the following: glucose, potato juice (powder), corn flour, agar (powder), a yeast extract (powder), wheat bran (husk), silkworm chrysalis powder, peptone, NaCl, $MgCl_2$ (magnesium chloride or its hydrates), $MgSO_4$ (magnesium sulfate or its hydrates), KCl, $CaCl_2$ (calcium chloride or its hydrates), and $KH_2PO_4$ (potassium dihydrogen phosphate or its hydrates). For example, the slant culture medium may include (the following % represents weight/volume ratio, g/100 mL):

0.05-2.0% of the peptone, 0.05-2.0% of the yeast extract, 1.0-5.0% of the glucose, 1.0-3.0% of the agar, 2.0-10% of the wheat bran, 0.02-0.2% of $KH_2PO_4$, 0.01-0.1% of $MgSO_4$, and the balance of water;
or
1.0-2.0% of the silkworm chrysalis powder, 1.0-2.0% of the corn flour, 2.0-5.0% of the glucose, 0.01-0.1% of $MgSO_4$, 0.01-0.1% of $KH_2PO_4$, 1.0-3.0 g/60 mL of oatmeal, 0.5-1% of the agar powder, and the balance of water;

or

1.8% of the silkworm chrysalis powder, 1.5% of the corn flour, 2.0% of the glucose, 0.01% of $MgSO_4$, 0.02% of $KH_2PO_4$, 1.0-3.0 g/60 mL of the oatmeal, 0.65% of the agar powder, and the balance of water;

or

0.1% of the peptone, 0.1% of the yeast extract, 2.0% of the glucose, 0.1% of $KH_2PO_4$, 0.05% of $MgSO_4$, 1.0-2.0% of the agar, and 5.0% of the wheat bran, which are boiled with water for 30 min, followed by separate packaging;

or

0.05-2.0% of the peptone, 2.0-5.0% of broad bean flour, 1.0-4.0% of the corn flour, 2.0-5.0% of the glucose, 0.5-2.0% of the agar, 0.5-4% of the wheat bran, 0.01-1.0% of $KH_2PO_4$, 0.01-1.0% of $MgSO_4$, and the balance of water;

or

2.0% of the glucose, 1.0% of the corn flour, 0.5% of the potato juice, 0.5% of dextrin, 0.5% of the yeast powder, 1.0% of the wheat bran, 2.0% of the silkworm chrysalis powder, 1.0% of the peptone, 0.05% of $MgSO_4$, 0.05% of $KH_2PO_4$, and 1.0% of agar powder, with water as a solvent;

or

2.0-3.0% of the silkworm chrysalis powder, 1.5% of the corn flour, 1.0-5.0% of the wheat bran, 2.0% of the glucose, 1.8% of the agar, 0.02% of $KH_2PO_4$, 0.01% of $MgSO_4$, and the balance of water.

Those skilled in the art will understand that the specific slant culture media listed above are provided merely to illustrate the present disclosure and should not be construed as limiting it. Furthermore, it will be understood by those skilled in the art that other common slant culture media for Hirsutella sinensis fermentation, or known slant culture media for Hirsutella sinensis fermentation in the prior art, may also be included.

In the present disclosure, for shake flask/seed flask culture, the strain is inoculated from the slant into a seed flask and then subjected to shake culture; or, the strain in a test tube is inoculated into a sterilized and cooled liquid medium in a triangular flask for shake culture on a shaker. For example, an inoculum size is 2-6 slants, preferably 2-3 slants; a culture temperature is 10-18°C, preferably 12-16°C; and a culture duration lasts for 8-16 d, preferably 9-14 d. In large-scale production, the mycelia cultured in shake flask can be inoculated into a seed tank to serve as a strain. In shake flask liquid culture, it requires rapid growth of the hyphae with a high yield and small mycelial pellets formed. If the mycelial pellets are too large, the mycelia at centers of the mycelial pellets will exhibit oxygen deficiency and nutrient starvation, which is unfavorable for hypha proliferation and production of secondary metabolites. This affects fermentation quality and the yield of the hyphae. The seed flask/shake flask culture medium includes the main raw material including one or more of the following: the peptone, fish peptone, a beef extract, the silkworm chrysalis powder, the glucose, the corn flour, the dextrin, the yeast extract (powder), the wheat bran (husk), NaCl, $MgCl_2$ (magnesium chloride or its hydrates), $MgSO_4$ (magnesium sulfate or its hydrates), KCl, $CaCl_2$ (calcium chloride or its hydrates), and $KH_2PO_4$ (potassium dihydrogen phosphate or its hydrates). For example, the shake flask/seed flask culture medium may include (the following % represents weight/volume ratio, g/100 mL):

2.0-3.0% of the silkworm chrysalis powder, 2.0-3.0% of the corn flour, 2.0-3.0% of the wheat bran, 0.1-0.5% of the fish peptone, 0.1-1% of the peptone, 0.01-0.1% of $KH_2PO_4$, 2.0-5.0% of the glucose, 0.01-0.1% of $MgSO_4$, and the balance of water;

or

2.0-3.0% of the silkworm chrysalis powder, 1.5% of the corn flour, 1.0-5.0% of the wheat bran, 2.0% of the glucose, 0.02% of $KH_2PO_4$, 0.01% of $MgSO_4$, and the balance of water;

or

2.0% of the silkworm chrysalis powder, 2.5% of the corn flour, 2.5% of the wheat bran, 0.25% of the fish peptone, 0.75% of the peptone, 0.02% of $KH_2PO_4$, 3.0% of the glucose, 0.01% of $MgSO_4$, and the balance of water;

or

2.0% of the glucose, 1.0% of the corn flour, 0.5% of dextrin, 0.5% of the yeast powder, 1.0% of the wheat bran, 2.0% of the silkworm chrysalis powder, 1.0% of the peptone, 0.05% of $MgSO_4$, and 0.05% of $KH_2PO_4$, with water as a solvent;

or

5.0% of the peptone, 2.0% of the glucose, 0.1% of $KH_2PO_4$, 0.05% of $MgSO_4$, and 5.0% of the wheat bran, with the water as the solvent;

or

0.5% of the fish peptone, 0.5% of the peptone, 2.0% of the glucose, 0.1% of $KH_2PO_4$, 0.05% of $MgSO_4$, 5% of the wheat bran, and the balance of water;

or

2.0-4.5% of the glucose, 2.0-4.5% of the beef extract; 1.0-2.5% of tomato powder, 1.0-2.5% of the peptone, 0.02-0.25% of $KH_2PO_4$, 0.01-0.05% of $MgSO_4$, 0.2-1.5% of inulin, 0.05-0.15% of vitamin B1, 0.01-0.06% of zinc acetate, and the balance of water.

Those skilled in the art will understand that the specific shake flask/seed flask culture media listed above are provided merely to illustrate the present disclosure and should not be construed as limiting it. Furthermore, it will be understood by those skilled in the art that other common shake flask/seed flask culture media for Hirsutella sinensis fermentation, or known shake flask/seed flask culture media for Hirsutella sinensis fermentation in the prior art, may also be included.

In the present disclosure, fermentation culture refers to a culture medium used for accumulation of a large amount of metabolites by microorganisms. Fermentation of the present disclosure involves use of a fermentation medium. Submerged fermentation refers to a method for culturing pure microbial cells in a liquid submerged medium under an aerobic or anaerobic condition. Submerged fermentation is typically carried out in a large-scale fermentation tank, enabling automated and mechanized production. A submerged fermentation process for Hirsutella sinensis in a fermentation tank can follow a workflow: primary seed tank → secondary seed tank → fermentation tank; alternatively, it can follow a workflow: seed tank → fermentation tank; or it can follow a continuous fermentation method. For example, a culture temperature is 10-18°C, preferably, 12-16°C; and a culture duration lasts for 6-20 d, preferably, 7-15 d, more preferably, 10-15 d. The fermentation medium includes the main raw materials including one or more of the following: the silkworm chrysalis powder, the glucose, the corn flour (slurry), the yeast extractum (extract paste or powder), NaCl, $MgCl_2$ (magnesium chloride or its hydrates), $MgSO_4$ (magnesium sulfate or its hydrates), KCl, $CaCl_2$ (calcium chloride or its hydrates), $KH_2PO_4$ (potassium dihydrogen phosphate or its hydrates), and polypeptide powder series (such as Polypeptide Powder 101, 102, 203, 305, 308, 607, 702, 705) (said polypeptide powder series is purchased from Chengdu Wenfeng Biotechnology Co., Ltd.).

The fermentation medium of the present disclosure involves use of a novel organic nitrogen source for microbial fermentation, such as the Polypeptide Powder "1" series. The Polypeptide Powder "1" series is primarily derived from rice protein, corn protein, and their hydrolysates. It is a powdered product produced through a multi-stage hydrolysis process using various hydrolysis methods, followed by separation, refined concentration, and drying. The Polypeptide Powder "1" series includes Polypeptide Powder 101 and Polypeptide Powder 102. The Polypeptide Powder "2" series, such as Polypeptide Powder 203, is primarily derived from the corn protein. It is produced through a multi-stage hydrolysis process using various hydrolysis methods (this process results in a product with varying molecular weights and reasonably distributed peptones and peptides, which facilitates continuous utilization by the microorganisms), followed by separation, refined concentration, and spray drying to prepare a powdered product.

The fermentation medium of the present disclosure involves use of a peptone series (non-animal-derived) from the novel organic nitrogen source for microbial fermentation, such as Polypeptide Powder 305, Polypeptide Powder 308, and Polypeptide Powder 607. This peptone series is primarily derived from the corn protein and a yeast. It is a powdered product produced through the multi-stage hydrolysis process using various hydrolysis methods (this process results in a product with varying molecular weights and reasonably distributed peptones and peptides, which facilitates continuous utilization by the microorganisms), followed by separation, refined concentration, and spray drying.

The fermentation medium of the present disclosure involves use of a peptone series (non-animal-derived) from the novel organic nitrogen source for microbial fermentation, such as Polypeptide Powder 702 and Polypeptide Powder 705. The Polypeptide Powder "7" series is primarily derived from the rice protein and the corn protein. It is a powdered product produced through a modern biological method combined with the multi-stage hydrolysis process using various hydrolysis methods (this process results in a product with varying molecular weights and reasonably distributed peptones and peptides, which facilitates continuous utilization by the microorganisms), followed by separation, refined concentration, and spray drying.

The fermentation medium of the present disclosure may include (the following % represents weight/volume ratio):

1-5% of the glucose, 0.5-5% of the yeast extract, 0.01-0.1% of $KH_2PO_4$, 0.01-0.1% of $MgSO_4$, 0.5-5% of the aforementioned polypeptide powder, and the balance of water;
preferably,
2.8% of the glucose, 1% of the yeast extract, 0.02% of $KH_2PO_4$, 0.01% of $MgSO_4$, 1% of the aforementioned polypeptide powder, and the balance of the water;
or,
2.8% of the glucose, 1% of the yeast extract, 0.02% of $KH_2PO_4$, 0.01% of $MgSO_4$, 1% of Polypeptide Powder 203, and the balance of the water;
2.8% of the glucose, 1% of the yeast extract, 0.02% of KH2PO4, 0.01% of $MgSO_4$, 1% of Polypeptide Powder 305, and the balance of the water;
2.8% of the glucose, 1% of the yeast extract, 0.02% of KH2PO4, 0.01% of $MgSO_4$, 1% of Polypeptide Powder 702, and the balance of the water;
2.8% of the glucose, 1% of the yeast extract, 0.02% of KH2PO4, 0.01% of $MgSO_4$, 1% of Polypeptide Powder 705, and the balance of the water;

2.8% of the glucose, 1% of the yeast extract, 0.02% of KH2PO4, 0.01% of $MgSO_4$, 1% of Polypeptide Powder 607, and the balance of the water.

The Cordyceps sinensis fermentation composition of the present disclosure refers to the mycelia, wet bacterial flakes, dried bacterial powder of the mycelia, or dried bacterial powder of the mycelia combined with a fermentation filtrate, obtained through slant culture, seed bottle/shake flask culture, and fermentation culture of Hirsutella sinensis, preferably, the dried bacterial powder of the mycelia, or the dried bacterial powder of the mycelia combined with the fermentation filtrate. The fermentation filtrate includes a fermentation filtrate obtained after solvent separation and chromatographic column separation (including membrane separation), as well as its effective fractions, effective components, or various metabolites.

In the present disclosure, through the optimized fermentation process, the obtained Cordyceps sinensis fermentation composition, mycelia and metabolites all have obviously increased component content, wherein the erythritol content is more than 1% (weight percentage), preferably, 1-6% (weight percentage), further preferably, 1.5-5% (weight percentage), 1.5-4% (weight percentage), 2-5% (weight percentage), more further preferably, 2.5-4% (weight percentage).

The present disclosure has unexpectedly found that more than 1% (weight percentage), preferably, 1-6% (weight percentage), further preferably, 1.5-5% (weight percentage), 1.5-4% (weight percentage), further preferably, 2-5% (weight percentage), more further preferably, 2.5-4% (weight percentage) of the erythritol in the Cordyceps sinensis fermentation composition is not only the first Cordyceps sinensis fermentation product with the stable erythritol content, but also enhances the effect of the adenosine. The inventor has unexpectedly found that combination of the erythritol and the adenosine shows an obvious synergistic effect on a hydroxyl radical scavenging rate. Therefore, on the other hand, when the present disclosure ensures that the erythritol of the obtained Cordyceps sinensis fermentation composition is sufficiently and stably present at more than 1% (weight percentage), preferably, 1-6% (weight percentage), further preferably, 1.5-5% (weight percentage), 1.5-4% (weight percentage), further preferably, 2-5% (weight percentage), more further preferably, 2.5-4% (weight percentage), the adenosine content is particularly preferred to be more than 0.02% (weight percentage), more than 0.03% (weight percentage), preferably, more than 0.03% (weight percentage), preferably, 0.03-1.00% (weight percentage), preferably, 0.03-0.80% (weight percentage), preferably, 0.03-0.60% (weight percentage), preferably, 0.03-0.40% (weight percentage), preferably, 0.03-0.20% (weight percentage), preferably, 0.03-0.10% (weight percentage), preferably, 0.03-0.08% (weight percentage).

Further, since previous studies have shown that the adenosine may be converted from the ATP, the adenosine diphosphate, and the adenosine monophosphate, but the nucleoside substances represented by the ATP, the adenosine diphosphate, and the adenosine monophosphate also have important physiological activity. As shown by Qian Jia et al. (Study on In Vitro Antioxidant Effect of 5'-acid Adenosine (5'-AMP), Food & Machinery, Volume 24, Issue 1, January 2008), the AMP has a strong activity of scavenging hydroxyl radicals. Therefore, on the basis of ensuring that the stable existence of the erythritol and the adenosine has a synergistic technical effect, the present disclosure controls the conversion amount of the adenosine to a certain extent, so that the Cordyceps sinensis fermentation composition as a whole shows a more excellent technical effect. Previous studies such as Chen Jiaming et al. (Effects of Different Carbon Sources and Nitrogen Sources on Solid Fermentation Process of Hirsutella sinensis, Edible Fungi of China 2017, 37(1): 55-60) showed that different carbon sources and nitrogen sources had an effect on the solid fermentation process of the Hirsutella sinensis. The highest adenosine content obtained by a solid fermentation method was only 63.76 $\mu g \cdot g^{-1}$ in formula G7. However, Zhang Ping et al. (Research Progress on Quality Evaluation and Control of Cordyceps sinensis Fermentation Products, Chinese Pharmaceutical Journal, Volume 56, Issue 14, July 2021) recorded that the culture conditions of the Hirsutella sinensis were optimized through liquid fermentation. After the optimization, the dry weight of the Hirsutella sinensis mycelium was increased by 36.2%, and the adenosine content was increased by 46.8%. During the fermentation process, amino nitrogen is the most important limiting matrix for cell growth. Therefore, obtaining the amino nitrogen during the liquefaction process is very important for increasing the yield of Cordyceps sinensis and effectively utilizing raw materials (CN103444434A). However, some studies have shown that the growth rate of cells is fastest when the concentrations of the carbon sources and nitrogen sources are low. As the concentrations of the carbon sources and the nitrogen sources increase, the growth rate of the cells decreases, indicating that higher concentrations of the carbon sources and nitrogen sources are not conducive to the cell growth (CN103430777A).

The present disclosure has unexpectedly found that the adenosine content is not as high as possible. On the basis that the Cordyceps sinensis fermentation composition of the present disclosure has the erythritol content of more than 1%, the adenosine content is preferably more than 0.02% (weight percentage), more than 0.03% (weight percentage), preferably, 0.03-1.00% (weight percentage), preferably, 0.03-0.80% (weight percentage), preferably, 0.03-0.60% (weight percentage), preferably, 0.03-0.40% (weight percentage), preferably, 0.03-0.20% (weight percentage), preferably, 0.03-0.10% (weight percentage), preferably, 0.03-0.08% (weight percentage).

More specifically, in the present disclosure, on the basis of the erythritol content of more than 1%, the adenosine content is preferably more than 0.02% (weight percentage); or,

more specifically, in the present disclosure, on the basis of the erythritol content of 1-6%, the adenosine content is preferably 0.02-1.00% (weight percentage); or,

more specifically, in the present disclosure, on the basis of the erythritol content of 1-6%, the adenosine content is preferably 0.03-0.80% (weight percentage); or,

more specifically, in the present disclosure, on the basis of the erythritol content of 1.5-5% (weight percentage), the adenosine content is preferably 0.02-1.00% (weight percentage); or,

more specifically, in the present disclosure, on the basis of the erythritol content of 1.5-4% (weight percentage), the adenosine content is preferably 0.02-1.00% (weight percentage); or,

more specifically, in the present disclosure, on the basis of the erythritol content of 2-5%, the adenosine content is preferably 0.02-1.00% (weight percentage); or,

more specifically, in the present disclosure, on the basis of the erythritol content of 2-5%, the adenosine content is preferably 0.03-0.80% or more (weight percentage); or,

more specifically, in the present disclosure, on the basis of the erythritol content of 2.5-4%, the adenosine content is preferably 0.02-1.00% (weight percentage).

More specifically, in the present disclosure, on the basis of the erythritol content of 2.5-4%, the adenosine content is preferably 0.03-0.80% (weight percentage).

Those skilled in the art may understand that the above illustrations are merely for explaining the present disclosure, rather than limiting the present application. With respect to the study on the erythritol content and the adenosine content, the fermentation composition obtained in the scope has an excellent antioxidant effect, particularly a synergistic effect on the hydroxyl radical scavenging rate.

Therefore, the present disclosure also provides a Cordyceps sinensis fermentation composition, further including one or more of the mannitol, the polysaccharides, and the amino acids.

Further, the Cordyceps sinensis fermentation composition has the mannitol content of more than 4% (weight percentage), preferably, more than 5% (weight percentage), preferably, more than 6% (weight percentage), preferably, more than 7% (weight percentage).

More specifically, in the present disclosure, on the basis of the erythritol content of more than 1%, the mannitol content is preferably more than 4% (weight percentage); or,

more specifically, in the present disclosure, on the basis of the erythritol content of 1%, the mannitol content is preferably more than 5% (weight percentage); or,

more specifically, in the present disclosure, on the basis of the erythritol content of 1%, the mannitol content is preferably more than 6% (weight percentage); or,

more specifically, in the present disclosure, on the basis of the erythritol content of 1%, the mannitol content is preferably more than 7% (weight percentage); or,

more specifically, in the present disclosure, on the basis of the erythritol content of 1-6%, the mannitol content is preferably more than 4% (weight percentage); or,

more specifically, in the present disclosure, on the basis of the erythritol content of 1-6%, the mannitol content is preferably more than 5% (weight percentage); or,

more specifically, in the present disclosure, on the basis of the erythritol content of 1-6%, the mannitol content is preferably more than 6% (weight percentage); or,

more specifically, in the present disclosure, on the basis of the erythritol content of 1-6%, the mannitol content is preferably more than 7% (weight percentage); or,

more specifically, in the present disclosure, on the basis of the erythritol content of 1.5-5%, the mannitol content is preferably more than 4% (weight percentage); or,

more specifically, in the present disclosure, on the basis of the erythritol content of 1.5-5%, the mannitol content is preferably more than 5% (weight percentage); or,

more specifically, in the present disclosure, on the basis of the erythritol content of 1.5-5%, the mannitol content is preferably more than 6% (weight percentage); or,

more specifically, in the present disclosure, on the basis of the erythritol content of 1.5-5%, the mannitol content is preferably more than 7% (weight percentage); or,

more specifically, in the present disclosure, on the basis of the erythritol content of 2-4%, the mannitol content is preferably more than 4% (weight percentage); or,

more specifically, in the present disclosure, on the basis of the erythritol content of 2-4%, the mannitol content is preferably more than 5% (weight percentage); or,

more specifically, in the present disclosure, on the basis of the erythritol content of 2-4%, the mannitol content is preferably more than 6% (weight percentage); or,

more specifically, in the present disclosure, on the basis of the erythritol content of 2-4%, the mannitol content is preferably more than 7% (weight percentage); or,

more specifically, in the present disclosure, on the basis of the erythritol content of 2.5-4%, the mannitol content is preferably more than 4% (weight percentage); or,

more specifically, in the present disclosure, on the basis of the erythritol content of 2.5-4%, the mannitol content is preferably more than 5% (weight percentage); or,

more specifically, in the present disclosure, on the basis of the erythritol content of 2.5-4%, the mannitol content is preferably more than 6% (weight percentage); or,

more specifically, in the present disclosure, on the basis of the erythritol content of 2.5-4%, the mannitol content is preferably more than 7% (weight percentage).

Further, the Cordyceps sinensis fermentation composition has the polysaccharide content of more than 2% (weight percentage), preferably, more than 3% (weight percentage), percentage), preferably, more than 4% (weight percentage), preferably, 2-10% (weight percentage).

Further, a total amino acid content is more than 20% (weight percentage), preferably, preferably 25-50% (weight percentage).

Those skilled in the art may understand that the above illustrations are merely for explaining the present disclosure, rather than limiting the present disclosure. With respect to studies on the erythritol, the adenosine content, the mannitol, and the polysaccharides, the fermentation composition obtained in the scope has excellent technical effects.

In another aspect, the present disclosure further provides the Cordyceps sinensis fermentation composition, having the erythritol content of more than 1% (weight percentage), preferably, 1-6% (weight percentage), further preferably, 1.5-5% (weight percentage), 1.5-4% (weight percentage), further preferably, 2-5% (weight percentage), more further preferably, 2.5-4% (weight percentage), having the adenosine content of more than 0.02% (weight percentage), and having the mannitol content of more than 4% (weight percentage).

Further, the Cordyceps sinensis fermentation composition in the present disclosure has the erythritol content of more than 1% (weight percentage), preferably, 1-6% (weight percentage), further preferably, 1.5-5% (weight percentage), 1.5-4% (weight percentage), further preferably, 2-5% (weight percentage), more further preferably, 2.5-4% (weight percentage), the adenosine content of more than 0.02% (weight percentage), the mannitol content of more than 4% (weight percentage), and the polysaccharide content of more than 2% (weight percentage).

Further, the Cordyceps sinensis fermentation composition has the erythritol content of more than 1% (weight percentage), preferably 1-6% (weight percentage), further preferably 1.5-5% (weight percentage), 1.5-4% (weight percentage), further preferably 2-5% (weight percentage), more further preferably 2.5-4% (weight percentage), the adenosine content of more than 0.02% (weight percentage), the mannitol content of more than 4% (weight percentage), and the total amino acid content of more than 20% (weight percentage), preferably 25-50% (weight percentage).

Further, the Cordyceps sinensis fermentation composition in the present disclosure has the erythritol content of more than 1% (weight percentage), preferably, 1-6% (weight percentage), further preferably, 1.5-5% (weight percentage), 1.5-4% (weight percentage), further preferably, 2-5% (weight percentage), more further preferably, 2.5-4% (weight percentage), the adenosine content of more than 0.02% (weight percentage), the mannitol content of more than 4% (weight percentage), the polysaccharide content of more than 2% (weight percentage), and the total amino acid content of more than 20% (weight percentage), preferably, 25-50% (weight percentage).

More specifically, the Cordyceps sinensis fermentation composition in the present disclosure has the erythritol content of more than 1% (weight percentage), preferably, 1-6% (weight percentage), further preferably, 1.5-5% (weight percentage), 1.5-4% (weight percentage), further preferably, 2-5% (weight percentage), more further preferably, 2.5-4% (weight percentage), the adenosine content, preferably, of more than 0.02% (weight percentage), and the mannitol content of more than 5% (weight percentage); or,

more specifically, the Cordyceps sinensis fermentation composition in the present disclosure has the erythritol content of more than 1% (weight percentage), preferably, 1-6% (weight percentage), further preferably, 1.5-5% (weight percentage), 1.5-4% (weight percentage), further preferably, 2-5% (weight percentage), more further preferably, 2.5-4% (weight percentage), the adenosine content, preferably, of more than 0.03% (weight percentage), and the mannitol content of more than 5% (weight percentage); or,

more specifically, the Cordyceps sinensis fermentation composition in the present disclosure has the erythritol content of more than 1% (weight percentage), preferably, 1-6% (weight percentage), further preferably, 1.5-5% (weight percentage), 1.5-4% (weight percentage), further preferably, 2-5% (weight percentage), more further preferably, 2.5-4% (weight percentage), the adenosine content, preferably, of more than 0.02% (weight percentage), the mannitol content of more than 5% (weight percentage), and the polysaccharide content of more than mounted 2% (weight percentage); or,

more specifically, the Cordyceps sinensis fermentation composition in the present disclosure has the erythritol content of more than 1% (weight percentage), preferably, 1-6% (weight percentage), further preferably, 1.5-5% (weight percentage), 1.5-4% (weight percentage), further preferably, 2-5% (weight percentage), more further preferably, 2.5-4% (weight percentage), the adenosine content, preferably, of more than 0.02% (weight percentage), the mannitol content of more than 5% (weight percentage), and the total amino acid content of more than 20% (weight percentage); or,

more specifically, the Cordyceps sinensis fermentation composition in the present disclosure has the erythritol content of more than 1% (weight percentage), preferably, 1-6% (weight percentage), further preferably, 1.5-5% (weight percentage), 1.5-4% (weight percentage), further preferably, 2-5% (weight percentage), more further preferably, 2.5-4% (weight percentage), the adenosine content, preferably, of more than 0.02-1.0% (weight percentage), and the mannitol content of more than 5% (weight percentage); or,

more specifically, the Cordyceps sinensis fermentation composition in the present disclosure has the erythritol content of more than 1% (weight percentage), preferably, 1-6% (weight percentage), further preferably, 1.5-5% (weight percentage), 1.5-4% (weight percentage), further preferably, 2-5% (weight percentage), more further preferably, 2.5-4% (weight percentage), the adenosine content, preferably, of more than 0.02-1.0% (weight percentage), the mannitol content of more than 5% (weight percentage), and the polysaccharide content of more than mounted 2% (weight percentage); or,

more specifically, the Cordyceps sinensis fermentation composition in the present disclosure has the erythritol content of more than 1% (weight percentage), preferably, 1-6% (weight percentage), further preferably, 1.5-5% (weight percentage), 1.5-4% (weight percentage), further preferably, 2-5% (weight percentage), more further preferably, 2.5-4% (weight percentage), the adenosine content, preferably, of more than 0.02-1.0% (weight percentage), the mannitol content of more than 5% (weight percentage), and the total amino acid content of more than 20% (weight percentage); or,

more specifically, the Cordyceps sinensis fermentation composition in the present disclosure has the erythritol content of more than 1% (weight percentage), preferably, 1-6% (weight percentage), further preferably, 1.5-5% (weight percentage), 1.5-4% (weight percentage), further preferably, 2-5% (weight percentage), more further preferably, 2.5-4% (weight percentage), the adenosine content, preferably, of more than 0.02-1.0% (weight percentage), the mannitol content of more than 5% (weight percentage), the polysaccharide content of more than 2% (weight percentage), and the total amino acid content of more than 20% (weight percentage).

The Cordyceps sinensis fermentation composition of the present disclosure can be directly prepared into various dosage forms, including powders, granules, tablets, pills, capsules, or by mixing the above components and packaging a mixture into capsules or tableting the mixture to obtain individual preparations. It can also be prepared into various dosage forms using acceptable carriers. With regard to orally administered compositions (for example, tablets and capsules), the term "acceptable carrier" includes such carriers as a conventional excipient, for example, an adhesive, such as syrup, acacia, gelatin, sorbitol, tragacanth, polyvinylpyrrolidone (povidone), methylcellulose, ethylcellulose, sodium carboxymethyl cellulose, hydroxypropyl methylcellulose, sucrose, and starch; a filler and a carrier such as corn starch, the gelatin, lactose, the sucrose, and microcrystalline cellulose; and a lubricant such as magnesium stearate, sodium stearate and other stearates, glyceryl monostearate, stearic acid, silicone fluid, talc, wax, oil, and colloidal silicon dioxide. A flavoring agent may also be used, for example, mint, wintergreen oil, and a cherry flavor. It may be necessary to add a coloring agent, in order to distinguish between dosage forms. The tablet may also be coated by a method well known in the art. The tablet may be compressed or molded, optionally including one or more auxiliary components. A compressed tablet may be such prepared by compressing a free-flowing form of the active components, such as the powders or the granules, in a suitable machine, optionally, mixed with one or more adhesives, lubricants, inert diluents, preservatives, surfactants, or dispersants.

In conclusion, the Cordyceps sinensis fermentation composition of the present disclosure is not only the first Cordyceps sinensis fermentation product with the stable erythritol content, but also, with respect to the study on the erythritol content and the adenosine content, the fermentation composition obtained in the scope has an excellent antioxidant effect, so that the Cordyceps sinensis fermentation composition as a whole shows the more excellent technical effect.

The drug fermentation composition of the present disclosure can be directly prepared into various dosage forms, including the powders, the granules, the tablets, the pills, the capsules, or by mixing the above components and packaging a mixture into the capsules or tableting the mixture to obtain individual preparations. It can also be prepared into various dosage forms using acceptable carriers.

The drug composition of the present disclosure has the excellent antioxidant effect and the synergistic technical effect.

In another aspect, the present disclosure obtains Cordyceps sinensis producing the ergothioneine by screening Cordyceps sinensis and researching Cordyceps sinensis fermentation and its beneficial components, and then obtains a fermentation composition including the ergothioneine through fermentation. That is, the present disclosure enables stable and sufficient presence of the ergothioneine in the Cordyceps sinensis mycelium or bacterial powder by employing the low-temperature liquid fermentation technology. The present disclosure achieves reasonable presence of effective components such as the ergothioneine and the adenosine through the low-temperature fermentation process. This not only enhances the stability of the Cordyceps sinensis fermentation composition, but also provides a new direction for utilizing fermented Cordyceps sinensis bacterial powder.

Specifically, in one aspect, for the present disclosure, the inventor has isolated Hirsutella sinensis capable of producing both the erythritol and the ergothioneine from natural Cordyceps sinensis by screening strains of Cordyceps sinensis. In another aspect, the inventor has obtained a stable fermented mycelium or bacterial powder with a high content of stably existing ergothioneine through fermentation of Hirsutella sinensis. Through liquid fermentation culture, the present disclosure achieves, for the first time, a fermentation composition with the ergothioneine at a content comparable to or higher than that found in natural Cordyceps sinensis, thereby enabling the Cordyceps sinensis fermentation composition to exert broader functionalities.

A Hirsutella sinensis strain is of an anamorphic generation of the fungus Cordyceps sinensis, which belongs to the family Clavieps purpurea (Fr.)Tul., and is isolated from fresh Cordyceps sinensis collected on the Qinghai-Tibet Plateau. Hirsutella sinensis described in the present disclosure may be any strain of Hirsutella sinensis. For example, it may be purchased from a commercial platform or obtained through independent screening and isolation, provided that it can be identified as Hirsutella sinensis following principles such as microbial molecular genetics. Commercial sources include, but are not limited to, China Center for Type Culture Collection (CCTCC) or China General Microbiological Culture Collection Center (CGMCC) for obtaining Hirsutella sinensis. Examples from the China Center for Type Culture Collection (CCTCC) include, but are not limited to, Hirsutella sinensis CCTCC No: M 2011278. Examples from the China General Microbiological Culture Collection Center (CGMCC) include, but are not limited to, Hirsutella sinensis CGMCC 3.14240 and Hirsutella sinensis CGMCC 3.14243. For example, standard strains of Ophiocordyceps sinensis (Hirsutella hepiali or Hirsutella sinensis) or Cordyceps sinensis (Hirsutella hepiali or Hirsutella sinensis) purchased from the China Center of Industrial Culture Collection (CICC) include, but are not limited to, strains with numbers: CICC 14016, CICC 14017, CICC 14088, CICC 14089, CICC 14090, CICC 14094, CICC 14096, CICC 14091, CICC 14092, CICC 14093, CICC 14095, CICC 14097, CICC 14098, CICC 14099, CICC 14100, CICC 14101, CICC 14102, CICC 14103, CICC 14104, CICC 14105, CICC 14106, CICC 14107, CICC 14108, CICC 14109, CICC 14110, CICC 14111, CICC 14112, CICC 14113, CICC 14114, CICC 14115, CICC 14117, CICC 14118, CICC 14119, CICC 14120, and CICC 50002. Examples of Ophiocordyceps sinensis (Hirsutella hepiali or Hirsutella sinensis), Cordyceps sinensis, or Hirsutella sinensis purchased from Tesuobio include, but are not limited to, products with codes: TS324838, TS325049, TS325054, TS326189, TS326195, TS326196, TS326197, TS326198, TS326199, TS326200, TS326201, TS326202, TS326203, TS326204, TS326205, TS326206, TS326207, TS326210, TS326212, TS326213, TS326214, TS326408, TS326410, TS326412, TS342791, TS342792, TS342793, TS342794, TS342795, TS342796, TS342797, TS342798, TS342800, TS342809, TS361737, and TS392986.

Hirsutella sinensis has a growth cycle longer than that of the vast majority of fungi. It typically grows in environments at 14-20°C and is classified as a mesophilic to low-temperature fungus. If Hirsutella sinensis is cultured on a solid medium, its diameter can reach up to 1.5 cm, and its colony is of a rubbery texture, appearing flesh-colored or milky-white. In an early stage of growth, aerial mycelia are present on the colony's surface. Over time, the colony's surface develops a smooth furrowed appearance, with the aerial mycelia gradually diminishing. If Hirsutella sinensis is cultured in a liquid medium, its mycelia appear as filaments or grey pellets in varying sizes. As culture proceeds, a brown secretion is released into the medium. The mycelial pellets are of a tough and unloosened texture, and are elastic and hollow inside. It is known that the mycelia obtained from liquid fermentation culture of Hirsutella sinensis is dried, and then becomes fermented Cordyceps sinensis bacterial powder. In this way, various active substances can be obtained, including polysaccharides, amino acids, nucleosides, sterols, mannitol, trace metals, etc. Among them, marker components include the adenosine, the mannitol, the polysaccharides, the amino acids, etc. The fermented Cordyceps sinensisbacterial powder exhibits various effects, including enhancing the immune function, exerting the anti-fibrosis activity, protecting the renal function, resisting the tumors and the inflammation, etc., and also provides the protective effects on the organs such as the kidney, the lung, and the liver.

Fermentation in the present disclosure refers to a process of culturing an existing strain of the anamorphic generation of Chinese Cordyceps sinensis, namely Hirsutella sinensis, with slant culture, seed flask/shake flask culture, and fermentation culture.

Slant culture of the present disclosure involves allowing Hirsutella sinensis to grow on a slant surface of a solid medium; and inoculating and spreading Hirsutella sinensis onto the slant surface for culture at a low temperature, for example, at 8-18°C for 20-60 d, preferably, at 10-16°C for 30-50 d, wherein a slant culture medium includes main components including one or more of the following: glucose, potato juice (powder), corn flour, agar (powder), a yeast extract (powder), wheat bran (husk), silkworm chrysalis powder, peptone, NaCl, $MgCl_2$ (magnesium chloride or its hydrates), $MgSO_4$ (magnesium sulfate or its hydrates), KCl, $CaCl_2$ (calcium chloride or its hydrates), and $KH_2PO_4$ (potassium dihydrogen phosphate or its hydrates). For example, the slant culture medium may include (the following % represents weight/volume ratio, g/100 mL):

0.05-2.0% of the peptone, 0.05-2.0% of the yeast extract, 1.0-5.0% of the glucose, 1.0-3.0% of the agar, 2.0-10% of the wheat bran, 0.02-0.2% of $KH_2PO_4$, 0.01-0.1% of $MgSO_4$, and the balance of water;

or

1.0-2.0% of the silkworm chrysalis powder, 1.0-2.0% of the corn flour, 2.0-5.0% of the glucose, 0.01-0.1% of $MgSO_4$, 0.01-0.1% of $KH_2PO_4$, 1.0-3.0 g/60 mL of oatmeal, 0.5-1% of the agar powder, and the balance of water;

or

1.8% of the silkworm chrysalis powder, 1.5% of the corn flour, 2.0% of the glucose, 0.01% of $MgSO_4$, 0.02% of $KH_2PO_4$, 1.0-3.0 g/60 mL of the oatmeal, 0.65% of the agar powder, and the balance of water;

or

0.1% of the peptone, 0.1% of the yeast extract, 2.0% of the glucose, 0.1% of $KH_2PO_4$, 0.05% of $MgSO_4$, 1.0-2.0% of the agar, and 5.0% of the wheat bran, which are boiled with water for 30 min, followed by separate packaging;

or

0.05-2.0% of the peptone, 2.0-5.0% of broad bean flour, 1.0-4.0% of the corn flour, 2.0-5.0% of the glucose, 0.5-2.0% of the agar, 0.5-4% of the wheat bran, 0.01-1.0% of $KH_2PO_4$, 0.01-1.0% of $MgSO_4$, and the balance of water;

or

2.0% of the glucose, 1.0% of the corn flour, 0.5% of the potato juice, 0.5% of dextrin, 0.5% of the yeast powder, 1.0% of the wheat bran, 2.0% of the silkworm chrysalis powder, 1.0% of the peptone, 0.05% of $MgSO_4$, 0.05% of $KH_2PO_4$, and 1.0% of agar powder, with water as a solvent;

or

2.0-3.0% of the silkworm chrysalis powder, 1.5% of the corn flour, 1.0-5.0% of the wheat bran, 2.0% of the glucose, 1.8% of the agar, 0.02% of $KH_2PO_4$, 0.01% of $MgSO_4$, and the balance of water.

Those skilled in the art will understand that the specific slant culture media listed above are provided merely to illustrate the present disclosure and should not be construed as limiting it. Furthermore, it will be understood by those skilled in the art that other common slant culture media for Hirsutella sinensis fermentation, or known slant culture media for Hirsutella sinensis fermentation in the prior art, may also be included.

In the present disclosure, for shake flask/seed flask culture, the strain is inoculated from the slant into a seed flask and then subjected to shake culture; or, the strain in a test tube is inoculated into a sterilized and cooled liquid medium in a triangular flask for shake culture on a shaker. For example, an inoculum size is 2-6 slants, preferably 2-3 slants; a culture temperature is 10-18°C, preferably 12-16°C; and a culture duration lasts for 8-16 d, preferably 9-14 d. In large-scale production, the mycelia cultured in shake flask can be inoculated into a seed tank to serve as a strain. In shake flask liquid culture, it requires rapid growth of the hyphae with a high yield and small mycelial pellets formed. If the mycelial pellets are too large, the mycelia at centers of the mycelial pellets will exhibit oxygen deficiency and nutrient starvation, which is unfavorable for hypha proliferation and production of secondary metabolites. This affects fermentation quality and the yield of the hyphae. The seed flask/shake flask culture medium includes the main raw material including one or more of the following: the peptone, the fish peptone, the beef extract, the silkworm chrysalis powder, the glucose, the corn flour, the dextrin, the yeast extract (powder), the wheat bran (husk), NaCl, $MgCl_2$ (magnesium chloride or its hydrates), $MgSO_4$ (magnesium sulfate or its hydrates), KCl, $CaCl_2$ (calcium chloride or its hydrates), and $KH_2PO_4$ (potassium dihydrogen phosphate or its hydrates). For example, the shake flask/seed flask culture medium may include (the following % represents weight/volume ratio, g/100 mL):

2.0-3.0% of the silkworm chrysalis powder, 2.0-3.0% of the corn flour, 2.0-3.0% of the wheat bran, 0.1-0.5% of the fish peptone, 0.1-1% of the peptone, 0.01-0.1% of $KH_2PO_4$, 2.0-5.0% of the glucose, 0.01-0.1% of $MgSO_4$, and the balance of water;

or

2.0-3.0% of the silkworm chrysalis powder, 1.5% of the corn flour, 1.0-5.0% of the wheat bran, 2.0% of the glucose, 0.02% of $KH_2PO_4$, 0.01% of $MgSO_4$, and the balance of water;

or

2.0% of the silkworm chrysalis powder, 2.5% of the corn flour, 2.5% of the wheat bran, 0.25% of the fish peptone, 0.75% of the peptone, 0.02% of $KH_2PO_4$, 3.0% of the glucose, 0.01% of $MgSO_4$, and the balance of water;

or

2.0% of the glucose, 1.0% of the corn flour, 0.5% of dextrin, 0.5% of the yeast powder, 1.0% of the wheat bran, 2.0% of the silkworm chrysalis powder, 1.0% of the peptone, 0.05% of $MgSO_4$, and 0.05% of $KH_2PO_4$, with water as a solvent;

or

5.0% of the peptone, 2.0% of the glucose, 0.1% of $KH_2PO_4$, 0.05% of $MgSO_4$, and 5.0% of the wheat bran, with the water as the solvent;

or

0.5% of the fish peptone, 0.5% of the peptone, 2.0% of the glucose, 0.1% of $KH_2PO_4$, 0.05% of $MgSO_4$, 5% of the

wheat bran, and the balance of water;
or
2.0-4.5% of the glucose, 2.0-4.5% of the beef extract; 1.0-2.5% of tomato powder, 1.0-2.5% of the peptone, 0.02-0.25% of $KH_2PO_4$, 0.01-0.05% of $MgSO_4$, 0.2-1.5% of inulin, 0.05-0.15% of vitamin B1, 0.01-0.06% of zinc acetate, and the balance of water.

Those skilled in the art will understand that the specific shake flask/seed flask culture media listed above are provided merely to illustrate the present disclosure and should not be construed as limiting it. Furthermore, it will be understood by those skilled in the art that other common shake flask/seed flask culture media for Hirsutella sinensis fermentation, or known shake flask/seed flask culture media for Hirsutella sinensis fermentation in the prior art, may also be included.

In the present disclosure, fermentation culture refers to a culture medium used for accumulation of a large amount of metabolites by microorganisms. Fermentation of the present disclosure involves use of a fermentation medium. Submerged fermentation refers to a method for culturing pure microbial cells in a liquid submerged medium under an aerobic or anaerobic condition. Submerged fermentation is typically carried out in a large-scale fermentation tank, enabling automated and mechanized production. A submerged fermentation process for Hirsutella sinensis in a fermentation tank can follow a workflow: primary seed tank → secondary seed tank → fermentation tank; alternatively, it can follow a workflow: seed tank → fermentation tank; or it can follow a continuous fermentation method. For example, a culture temperature is 10-18°C, preferably, 12-16°C; and a culture duration lasts for 6-20 d, preferably, 7-15 d, more preferably, 10-15 d. The fermentation medium includes the main raw materials including one or more of the following: the silkworm chrysalis powder, the glucose, the corn flour (slurry), the yeast extractum (extract paste or powder), NaCl, $MgCl_2$ (magnesium chloride or its hydrates), $MgSO_4$ (magnesium sulfate or its hydrates), KCl, $CaCl_2$ (calcium chloride or its hydrates), $KH_2PO_4$ (potassium dihydrogen phosphate or its hydrates), and polypeptide powder series (such as Polypeptide Powder 101, 102, 203, 305, 308, 607, 702, 705) (said polypeptide powder series is purchased from Chengdu Wenfeng Biotechnology Co., Ltd.).

The fermentation medium of the present disclosure involves use of a novel organic nitrogen source for microbial fermentation, such as the Polypeptide Powder "1" series. The Polypeptide Powder "1" series is primarily derived from rice protein, corn protein, and their hydrolysates. It is a powdered product produced through a multi-stage hydrolysis process using various hydrolysis methods, followed by separation, refined concentration, and drying. The Polypeptide Powder "1" series includes Polypeptide Powder 101 and Polypeptide Powder 102. The Polypeptide Powder "2" series, such as Polypeptide Powder 203, is primarily derived from the corn protein. It is produced through a multi-stage hydrolysis process using various hydrolysis methods (this process results in a product with varying molecular weights and reasonably distributed peptones and peptides, which facilitates continuous utilization by the microorganisms), followed by separation, refined concentration, and spray drying to prepare a powdered product.

The fermentation medium of the present disclosure involves use of a peptone series (non-animal-derived) from the novel organic nitrogen source for microbial fermentation, such as Polypeptide Powder 305, Polypeptide Powder 308, and Polypeptide Powder 607. This peptone series is primarily derived from the corn protein and a yeast. It is a powdered product produced through the multi-stage hydrolysis process using various hydrolysis methods (this process results in a product with varying molecular weights and reasonably distributed peptones and peptides, which facilitates continuous utilization by the microorganisms), followed by separation, refined concentration, and spray drying.

The fermentation medium of the present disclosure involves use of a peptone series (non-animal-derived) from the novel organic nitrogen source for microbial fermentation, such as Polypeptide Powder 702 and Polypeptide Powder 705. The Polypeptide Powder "7" series is primarily derived from the rice protein and the corn protein. It is a powdered product produced through a modern biological method combined with the multi-stage hydrolysis process using various hydrolysis methods (this process results in a product with varying molecular weights and reasonably distributed peptones and peptides, which facilitates continuous utilization by the microorganisms), followed by separation, refined concentration, and spray drying.

The fermentation medium of the present disclosure may include (the following % represents weight/volume ratio):

1-5% of the glucose, 0.5-5% of the yeast extract, 0.01-0.1% of $KH_2PO_4$, 0.01-0.1% of $MgSO_4$, 0.5-5% of the aforementioned polypeptide powder, and the balance of water;
preferably,
2.8% of the glucose, 1% of the yeast extract, 0.02% of $KH_2PO_4$, 0.01% of $MgSO_4$, 1% of the aforementioned polypeptide powder, and the balance of the water;
or,
2.8% of the glucose, 1% of the yeast extract, 0.02% of $KH_2PO_4$, 0.01% of $MgSO_4$, 1% of Polypeptide Powder 203, and the balance of the water;

2.8% of the glucose, 1% of the yeast extract, 0.02% of $KH_2PO_4$, 0.01% of $MgSO_4$, 1% of Polypeptide Powder 305, and the balance of the water;

2.8% of the glucose, 1% of the yeast extract, 0.02% of $KH_2PO_4$, 0.01% of $MgSO_4$, 1% of Polypeptide Powder 702, and the balance of the water;

2.8% of the glucose, 1% of the yeast extract, 0.02% of $KH_2PO_4$, 0.01% of $MgSO_4$, 1% of Polypeptide Powder 705, and the balance of the water;

2.8% of the glucose, 1% of the yeast extract, 0.02% of $KH_2PO_4$, 0.01% of $MgSO_4$, 1% of Polypeptide Powder 607, and the balance of the water.

The Cordyceps sinensis fermentation composition of the present disclosure refers to the mycelia, wet bacterial flakes, dried bacterial powder of the mycelia, or dried bacterial powder of the mycelia combined with a fermentation filtrate, obtained through slant culture, seed bottle/shake flask culture, and fermentation culture of Hirsutella sinensis, preferably, the dried bacterial powder of the mycelia, or the dried bacterial powder of the mycelia combined with the fermentation filtrate. The fermentation filtrate includes a fermentation filtrate obtained after solvent separation and chromatographic column separation (including membrane separation), as well as its effective fractions, effective components, or various metabolites.

In the present disclosure, through the optimized fermentation process, the obtained Cordyceps sinensis fermentation composition, mycelia and metabolites all have obviously increased component contents, wherein the ergothioneine content is more than 0.004% (weight percentage), preferably, more than 0.006% (weight percentage), preferably, more than 0.008% (weight percentage), preferably, more than 0.01% (weight percentage), preferably, 0.01-0.10% (weight percentage), preferably, 0.01-0.08% (weight percentage), or preferably, more than 0.02% (weight percentage), preferably, 0.02-0.10% (weight percentage), preferably, 0.02-0.08% (weight percentage); and the adenosine content is more than 0.02% (weight percentage), more than 0.03% (weight percentage), preferably, 0.03-1.00% (weight percentage), preferably, 0.03-0.80% (weight percentage), preferably, 0.03-0.60% (weight percentage), preferably, 0.03-0.40% (weight percentage), preferably, 0.03-0.20% (weight percentage), preferably, 0.03-0.10% (weight percentage), preferably, 0.03-0.08% (weight percentage).

A ratio of the ergothioneine content to the adenosine content is 1:1-1:20, preferably, 1:1-1:15, preferably, 1:1-1:10, preferably, 1:1-1:8, preferably, 1:1-1:7, preferably, 1:1-1:6, preferably, 1:1-1:5, preferably, 1:1-1:4, preferably, 1:1-1:3, preferably, 1:1-1:2.

Further, the Cordyceps sinensis fermentation composition has the erythritol content of more than 1% (weight percentage), preferably 1-6% (weight percentage), further preferably 1.5-5% (weight percentage), 1.5-4% (weight percentage), further preferably 2-5% (weight percentage), more further preferably 2.5-4% (weight percentage).

Further, the Cordyceps sinensis fermentation composition has the mannitol content of more than 4% (weight percentage), preferably, more than 5% (weight percentage), preferably, more than 6% (weight percentage), preferably, more than 7% (weight percentage); and the ratio of the ergothioneine content to the adenosine content is 1:1-1:1200, preferably, 1:1-1:1100, preferably, 1:1-1:1000, preferably, 1:1-1:900, preferably, 1:1-1:800, preferably, 1:1-1:700, preferably, 1:1-1:600, preferably, 1:1-1:500, preferably, 1:1-1:400, preferably, 1:1-1:300.

Further, the Cordyceps sinensis fermentation composition has the polysaccharide content of more than 2% (weight percentage), preferably, more than 3% (weight percentage), percentage), preferably, more than 4% (weight percentage), preferably, 2-10% (weight percentage); and the ratio of the ergothioneine content to the polysaccharide content is 1:1-1:500, preferably, 1:1-1:400, preferably, 1:1-1:300, preferably, 1:1-1:200, preferably, 1:1-1:100, preferably, 1:1-1:50.

Further, the total amino acid content is more than 20% (weight percentage), preferably, preferably 25-50% (weight percentage).

When the components contained in the Cordyceps sinensis fermentation composition provided by the present disclosure are within the above preferred range, the fermentation composition has excellent antioxidant and whitening effects as a whole.

Therefore, on the one hand, the present disclosure achieves that ergothioneine is sufficiently and stably present in Cordyceps sinensis mycelium or bacterial powder by a low-temperature fermentation technology, which is at least more than 0.004% (weight percentage).

Ergothioneine (EGT) is a white, odorless, colorless crystalline powder in a solid state. In this solid state, only the thione form exists. The EGT is easily soluble in water and has tautomers (thione and thiol forms) in the solution. However, the EGT has a very high thermal stability, and the decomposition temperature thereof is as high as 262-265°C. The EGT cannot be synthesized by the animal body itself but can only be obtained from food, and is a rare amino acid. Mammals and humans mainly obtain the EGT through diet. The EGT can be highly absorbed by the human body after ingestion. The EGT has strong oxidation resistance and plays a unique role in the body's cell synthesis process. The EGT has a good effect in preventing diabetes, kidney diseases, tissue fibrosis, eye diseases and cancer, resisting ultraviolet damage, inflammation and oxidation, promoting neuronal differentiation, and enhancing memory and learning ability. The European Food Safety Authority and the U.S. Food and Drug Administration have both affirmed the important position of the EGT in new functional foods and recognized the safety of producing the EGT through fermentation, and even believe that the EGT can be added to the diet of pregnant women and maternal and infant food. The oxidation resistance of the EGT is related to at least four molecular activities: direct scavenging of reactive oxygen species; chelation of various divalent metal cations; activation of antioxidant enzymes such as glutathione peroxidase and manganese superoxide dismutase (SOD), and inhibition of superoxidative kinases such as cytochrome C reductase (NADPH); and influence on oxidation of various hemoproteins such as heme and myoglobin.

The present disclosure has unexpectedly found that more than 0.004% (weight percentage), preferably, more than 0.006% (weight percentage), preferably, more than 0.008% (weight percentage), preferably, more than 0.01% (weight percentage) of the EGT in the Cordyceps sinensis fermentation composition is not only the first Cordyceps sinensis fermentation product with the EGT, but also greatly enhances the effect of adenosine. The present disclosure shows through research experiments that the EGT in the Cordyceps sinensis fermentation composition helps to enhance the antioxidant effect of the adenosine, so that the EGT and the adenosine produce a synergistic effect, and through the optimization of an adenosine content range, the inventor unexpectedly found that a Hirsutella sinensis fermentation product or the fermentation composition as a whole shows a more excellent technical effect.

Therefore, on the other hand, when the present disclosure ensures that the EGT of the obtained Cordyceps sinensis fermentation composition is sufficiently and stably present at more than 0.004% (weight percentage), preferably, more than 0.006% (weight percentage), preferably, more than 0.008% (weight percentage), preferably, more than 0.01% (weight percentage), the adenosine content is particularly preferred to be more than 0.02% (weight percentage), more than 0.03% (weight percentage), preferably, 0.03-1.00% (weight percentage), preferably, 0.03-0.80% (weight percentage), preferably, 0.03-0.60% (weight percentage), preferably, 0.03-0.40% (weight percentage), preferably, 0.03-0.20% (weight percentage), preferably, 0.03-0.10% (weight percentage), preferably, 0.03-0.08% (weight percentage); and the ratio of the EGT to the adenosine content is 1:1-1:20, preferably, 1:1-1:15, preferably, 1:1-1:10, preferably, 1:1-1:8, preferably, 1:1-1:7, preferably, 1:1-1:6, preferably, 1:1-1:5, preferably, 1:1-1:4, preferably, 1:1-1:3, preferably, 1:1-1:2.

The adenosine is slightly soluble in cold water, soluble in normal temperature water, easily soluble in hot water, and insoluble in alcohol. Studies have shown that, such as Li Heyu et al. (Determination of Five Nucleoside Components in Cordyceps sinensis. Products and Quality Analysis of Cordyceps militaris, Chinese Herbal Medicines, 2018(49) 22:5410-5417), guanosine, uridine and adenosine may be converted in the extraction process. For example, adenosine triphosphate (ATP), adenosine diphosphate and adenosine monophosphate can be converted into adenosine during ultrasonic extraction using water or mixed reagents with high water content. For example, Qian Zhengming et al. (Study on Transformation Pathway of Adenosine During Water Extraction of Cordyceps sinensis, World Journal of Traditional Chinese Medicine, 2016(11)5: 758-762) found that under the room temperature water extraction of Cordyceps sinensis, the adenosine monophosphate was converted into the adenosine, and the adenosine was converted into inosine. It was also found that under the room temperature water extraction condition, the ATP and the adenosine diphosphate were also converted into the adenosine. Wherein, the ATP is highly soluble in water and is very stable in solutions between pH 6.8 and 7.4, but will be rapidly hydrolyzed at extreme pH values. The adenosine is active in cardiovascular diseases. For example, Feng Yanhong et al. (Effects of Adenosine on Isolated Heart Functions and Myocardial Cell Apoptosis in Rats, Shandong Medicine Journal, Volume 53, Issue 46, 2013) showed that the SOD is the most important antioxidant enzyme, which can effectively scavenge excess free radicals in the body. The SOD activity index in a myocardial tissue of an adenosine group was better than that of an ischemia-reperfusion group.

The present disclosure has unexpectedly found that more than 0.004% (weight percentage), preferably, more than 0.006% (weight percentage), preferably, more than 0.008% (weight percentage), preferably, more than 0.01% (weight percentage) of the EGT in the Cordyceps sinensis fermentation composition is not only the first Cordyceps sinensis fermentation product with the EGT, but also enhances the effect of the adenosine. The inventor unexpectedly found that the combination of the ergothioneine and the adenosine shows an obvious synergistic effect on hydroxyl radical scavenging activity. Therefore, on the other hand, when the present disclosure ensures that the EGT of the obtained Cordyceps sinensis fermentation composition is sufficiently and stably present at more than 0.004% (weight percentage), preferably, more than 0.006% (weight percentage), preferably, more than 0.008% (weight percentage), preferably, more than 0.01% (weight percentage), the adenosine content is particularly preferred to be more than 0.02% (weight percentage), more than 0.03% (weight percentage), preferably, more than 0.03% (weight percentage), preferably, 0.03-1.00% (weight percentage), preferably, 0.03-0.80% (weight percentage), preferably, 0.03-0.60% (weight percentage), preferably, 0.03-0.40% (weight percentage), preferably, 0.03-0.20% (weight percentage), preferably, 0.03-0.10% (weight percentage), preferably, 0.03-0.08% (weight percentage); the ratio of the EGT to the adenosine content is 1:1-1:20, preferably, 1:1-1:15, preferably, 1:1-1:10, preferably, 1:1-1:8, preferably, 1:1-1:7, preferably, 1:1-1:6, preferably, 1:1-1:5, preferably, 1:1-1:4, preferably, 1:1-1:3, preferably, 1:1-1:2.

Further, since previous studies have shown that the adenosine may be converted from the ATP, the adenosine diphosphate, and the adenosine monophosphate, but the nucleoside substances represented by the ATP, the adenosine diphosphate, and the adenosine monophosphate also have important physiological activity. As shown by Qian Jia et al.

(Study on In Vitro Antioxidant Effect of 5'-acid Adenosine (5'-AMP), Food & Machinery, Volume 24, Issue 1, January 2008), the AMP has a strong activity of scavenging hydroxyl radicals. Therefore, on the basis of ensuring that the stable existence of the EGT and the adenosine has a synergistic technical effect, the present disclosure controls the conversion amount of the adenosine to a certain extent, so that the Cordyceps sinensis fermentation composition as a whole shows a more excellent technical effect. Previous studies such as Chen Jiaming et al. (Effects of Different Carbon Sources and Nitrogen Sources on Solid Fermentation Process of Hirsutella sinensis, Edible Fungi of China 2017, 37(1): 55-60) showed that different carbon sources and nitrogen sources had an effect on the solid fermentation process of the Hirsutella sinensis. The highest adenosine content obtained by a solid fermentation method was only 63.76 $\mu g \cdot g^{-1}$ in formula G7. However, Zhang Ping et al. (Research Progress on Quality Evaluation and Control of Cordyceps sinensis Fermentation Products, Chinese Pharmaceutical Journal, Volume 56, Issue 14, July 2021) recorded that the culture conditions of the Hirsutella sinensis were optimized through liquid fermentation. After the optimization, the dry weight of the Hirsutella sinensis mycelium was increased by 36.2%, and the adenosine content was increased by 46.8%. During the fermentation process, amino nitrogen is the most important limiting matrix for cell growth. Therefore, obtaining the amino nitrogen during the liquefaction process is very important for increasing the yield of Cordyceps sinensis and effectively utilizing raw materials (CN103444434A). However, some studies have shown that the growth rate of cells is fastest when the concentrations of the carbon sources and nitrogen sources are low. As the concentrations of the carbon sources and the nitrogen sources increases, the growth rate of the cells decreases, indicating that higher concentrations of the carbon sources and nitrogen sources are not conducive to the cell growth (CN103430777A).

The present disclosure has unexpectedly found that the adenosine content is not as high as possible. On the basis that the Cordyceps sinensis fermentation composition of the present disclosure has an EGT content of more than 0.004%, the adenosine content is preferably more than 0.02% (weight percentage), more than 0.03% (weight percentage), preferably, 0.03-1.00% (weight percentage), preferably, 0.03-0.80% (weight percentage), preferably, 0.03-0.60% (weight percentage), preferably, 0.03-0.40% (weight percentage), preferably, 0.03-0.20% (weight percentage), preferably, 0.03-0.10% (weight percentage), preferably, 0.03-0.08% (weight percentage); and the ratio of the EGT to the adenosine content is 1:1-1:20, preferably, 1:1-1:15, preferably, 1:1-1:10, preferably, 1:1-1:8, preferably, 1:1-1:7, preferably, 1:1-1:6, preferably, 1:1-1:5, preferably, 1:1-1:4, preferably, 1:1-1:3, preferably, 1:1-1:2. In the present disclosure, when the content of both the adenosine and the EGT is within the above preferred range, the Cordyceps sinensis fermentation composition as a whole can have the excellent antioxidant effect, which satisfies the application requirements well.

More specifically, in the present disclosure, on the basis of the EGT content of more than 0.01%, preferably the adenosine content of more than 0.02% (weight percentage), the ratio of the EGT content to the adenosine content is 1:1-1:20; or,

more specifically, in the present disclosure, on the basis of the EGT content of more than 0.01%, preferably the adenosine content of more than 0.02% (weight percentage), the ratio of the EGT content to the adenosine content is 1:1-1:15; or,
more specifically, in the present disclosure, on the basis of the EGT content of more than 0.01%, preferably the adenosine content of more than 0.02% (weight percentage), the ratio of the EGT content to the adenosine content is 1:1-1:10; or,
more specifically, in the present disclosure, on the basis of the EGT content of more than 0.01%, preferably the adenosine content of more than 0.02% (weight percentage), the ratio of the EGT content to the adenosine content is 1:1-1:8; or,
more specifically, in the present disclosure, on the basis of the EGT content of more than 0.01%, preferably the adenosine content of more than 0.02% (weight percentage), the ratio of the EGT content to the adenosine content is 1:1-1:7; or,
more specifically, in the present disclosure, on the basis of the EGT content of more than 0.01%, preferably the adenosine content of more than 0.02% (weight percentage), the ratio of the EGT content to the adenosine content is 1:1-1:6; or,
more specifically, in the present disclosure, on the basis of the EGT content of more than 0.01%, preferably the adenosine content of more than 0.02% (weight percentage), the ratio of the EGT content to the adenosine content is 1:1-1:5; or,
more specifically, in the present disclosure, on the basis of the EGT content of more than 0.01%, preferably the adenosine content of more than 0.02% (weight percentage), the ratio of the EGT content to the adenosine content is 1:1-1:4; or,
more specifically, in the present disclosure, on the basis of the EGT content of more than 0.01%, preferably the adenosine content of more than 0.02% (weight percentage), the ratio of the EGT content to the adenosine content is 1:1-1:3; or,
more specifically, in the present disclosure, on the basis of the EGT content of more than 0.01%, preferably the adenosine content of more than 0.02% (weight percentage), the ratio of the EGT content to the adenosine content is 1:1-1:2; or,

more specifically, in the present disclosure, on the basis of the EGT content of more than 0.01%, preferably the adenosine content of 0.03-0.08% (weight percentage), the ratio of the EGT content to the adenosine content is 1:1-1:20; or,

more specifically, in the present disclosure, on the basis of the EGT content of more than 0.01%, preferably the adenosine content of 0.03-0.08% (weight percentage), the ratio of the EGT content to the adenosine content is 1:1-1:15; or,

more specifically, in the present disclosure, on the basis of the EGT content of more than 0.01%, preferably the adenosine content of 0.03-0.08% (weight percentage), the ratio of the EGT content to the adenosine content is 1:1-1:10; or,

more specifically, in the present disclosure, on the basis of the EGT content of more than 0.01%, preferably the adenosine content of 0.03-0.08% (weight percentage), the ratio of the EGT content to the adenosine content is 1:1-1:8; or,

more specifically, in the present disclosure, on the basis of the EGT content of more than 0.01%, preferably the adenosine content of 0.03-0.08% (weight percentage), the ratio of the EGT content to the adenosine content is 1:1-1:7; or,

more specifically, in the present disclosure, on the basis of the EGT content of more than 0.01%, preferably the adenosine content of 0.03-0.08% (weight percentage), the ratio of the EGT content to the adenosine content is 1:1-1:6; or,

more specifically, in the present disclosure, on the basis of the EGT content of more than 0.01%, preferably the adenosine content of 0.03-0.08% (weight percentage), the ratio of the EGT content to the adenosine content is 1:1-1:5; or,

more specifically, in the present disclosure, on the basis of the EGT content of more than 0.01%, preferably the adenosine content of 0.03-0.08% (weight percentage), the ratio of the EGT content to the adenosine content is 1:1-1:4; or,

more specifically, in the present disclosure, on the basis of the EGT content of more than 0.01%, preferably the adenosine content of 0.03-0.08% (weight percentage), the ratio of the EGT content to the adenosine content is 1:1-1:3; or,

more specifically, in the present disclosure, on the basis of the EGT content of more than 0.01%, preferably the adenosine content of 0.03-0.08% (weight percentage), the ratio of the EGT content to the adenosine content is 1:1-1:2; or,

more specifically, in the present disclosure, on the basis of the EGT content of more than 0.02%, preferably the adenosine content of more than 0.02% (weight percentage), the ratio of the EGT content to the adenosine content is 1:1-1:20; or,

more specifically, in the present disclosure, on the basis of the EGT content of more than 0.02%, preferably the adenosine content of more than 0.02% (weight percentage), the ratio of the EGT content to the adenosine content is 1:1-1:15; or,

more specifically, in the present disclosure, on the basis of the EGT content of more than 0.02%, preferably the adenosine content of more than 0.02% (weight percentage), the ratio of the EGT content to the adenosine content is 1:1-1:10; or,

more specifically, in the present disclosure, on the basis of the EGT content of more than 0.02%, preferably the adenosine content of more than 0.02% (weight percentage), the ratio of the EGT content to the adenosine content is 1:1-1:8; or,

more specifically, in the present disclosure, on the basis of the EGT content of more than 0.02%, preferably the adenosine content of more than 0.02% (weight percentage), the ratio of the EGT content to the adenosine content is 1:1-1:7; or,

more specifically, in the present disclosure, on the basis of the EGT content of more than 0.02%, preferably the adenosine content of more than 0.02% (weight percentage), the ratio of the EGT content to the adenosine content is 1:1-1:6; or,

more specifically, in the present disclosure, on the basis of the EGT content of more than 0.02%, preferably the adenosine content of more than 0.02% (weight percentage), the ratio of the EGT content to the adenosine content is 1:1-1:5; or,

more specifically, in the present disclosure, on the basis of the EGT content of more than 0.02%, preferably the adenosine content of more than 0.02% (weight percentage), the ratio of the EGT content to the adenosine content is 1:1-1:4; or,

more specifically, in the present disclosure, on the basis of the EGT content of more than 0.02%, preferably the adenosine content of more than 0.02% (weight percentage), the ratio of the EGT content to the adenosine content is 1:1-1:3; or,

more specifically, in the present disclosure, on the basis of the EGT content of more than 0.02%, preferably the adenosine content of more than 0.02% (weight percentage), the ratio of the EGT content to the adenosine content is 1:1-1:2; or,

more specifically, in the present disclosure, on the basis of the EGT content of more than 0.02%, preferably the adenosine content of 0.03-0.08% (weight percentage), the ratio of the EGT content to the adenosine content is 1:1-1:20; or,

more specifically, in the present disclosure, on the basis of the EGT content of more than 0.02%, preferably the adenosine content of 0.03-0.08% (weight percentage), the ratio of the EGT content to the adenosine content is 1:1-1:15; or,

more specifically, in the present disclosure, on the basis of the EGT content of more than 0.02%, preferably the adenosine content of 0.03-0.08% (weight percentage), the ratio of the EGT content to the adenosine content is 1:1-1:10; or,

more specifically, in the present disclosure, on the basis of the EGT content of more than 0.02%, preferably the adenosine content of 0.03-0.08% (weight percentage), the ratio of the EGT content to the adenosine content is 1:1-1:8; or,

more specifically, in the present disclosure, on the basis of the EGT content of more than 0.02%, preferably the adenosine content of 0.03-0.08% (weight percentage), the ratio of the EGT content to the adenosine content is 1:1-1:7; or,

more specifically, in the present disclosure, on the basis of the EGT content of more than 0.02%, preferably the adenosine content of 0.03-0.08% (weight percentage), the ratio of the EGT content to the adenosine content is 1:1-1:6; or,

more specifically, in the present disclosure, on the basis of the EGT content of more than 0.02%, preferably the adenosine content of 0.03-0.08% (weight percentage), the ratio of the EGT content to the adenosine content is 1:1-1:5; or,

more specifically, in the present disclosure, on the basis of the EGT content of more than 0.02%, preferably the adenosine content of 0.03-0.08% (weight percentage), the ratio of the EGT content to the adenosine content is 1:1-1:4; or,

more specifically, in the present disclosure, on the basis of the EGT content of more than 0.02%, preferably the adenosine content of 0.03-0.08% (weight percentage), the ratio of the EGT content to the adenosine content is 1:1-1:3; or,

more specifically, in the present disclosure, on the basis of the EGT content of more than 0.02%, preferably the adenosine content of 0.03-0.08% (weight percentage), the ratio of the EGT content to the adenosine content is 1:1-1:2.

Those skilled in the art may understand that the above illustrations are merely for explaining the present disclosure, rather than limiting the present application. With respect to the study on the EGT content and the adenosine content, the fermentation composition obtained in the scope has an excellent antioxidant effect, particularly a synergistic effect on the hydroxyl radical scavenging rate.

In another aspect of the present disclosure, in combination with the conversion behavior of the EGT content and the adenosine, on the basis of the content of the EGT, erythritol and adenosine, the yield of dry weight of the mycelia is further researched, that is, on the basis of ensuring the content of the EGT, erythritol and adenosine, the Cordyceps sinensis fermentation composition having higher mycelium yield is further obtained by the inventor by optimizing the fermentation process. The mycelium fermentation yield is up to 7 g/L or more, preferably 7-15 g/L, 7-20 g/L, 8-15 g/L and 8-20 g/L. The water content of the dry weight of the mycelia is 8% or less, preferably the water content of 7% or less, more preferably the water content of 6% or less.

More specifically, according to the Cordyceps sinensis fermentation composition in the present disclosure, on the basis of the EGT content of more than 0.01%, preferably the adenosine content of more than 0.02% (weight percentage), the ratio of the EGT content to the adenosine content is 1:1-1:20, and the water content of the dry weight of the mycelia is 7% or less; or,

more specifically, according to the Cordyceps sinensis fermentation composition in the present disclosure, on the basis of the EGT content of more than 0.01%, preferably the adenosine content of more than 0.02% (weight percentage), the ratio of the EGT content to the adenosine content is 1:1-1:20, and the mycelium fermentation yield is 7 g/L or more; or,

more specifically, according to the Cordyceps sinensis fermentation composition in the present disclosure, on the basis of the EGT content of more than 0.01%, preferably the adenosine content of 0.02-0.2% (weight percentage), the ratio of the EGT content to the adenosine content is 1:1-1:20, and the water content of the dry weight of the mycelia is 7% or less; or,

more specifically, according to the Cordyceps sinensis fermentation composition in the present disclosure, on the basis of the EGT content of more than 0.01%, preferably the adenosine content of 0.02-0.2% (weight percentage), the ratio of the EGT content to the adenosine content is 1:1-1:20, and the mycelium fermentation yield is 7 g/L or more.

Further, through an in-depth study of the inventor, on the basis of the EGT content of more than 0.004% (weight percentage), preferably more than 0.006% (weight percentage), preferably more than 0.008% (weight percentage), preferably more than 0.01%, it is accidentally discovered in the present disclosure that, due to existence of the EGT content of more than 0.004%, the Cordyceps sinensis fermentation composition is the first Cordyceps sinensis. fermentation product having the EGT, and significantly enhances effects of other beneficial components. Through research experiments in the present disclosure, the EGT in the Cordyceps sinensis fermentation composition contributes to enhancing the effects of the adenosine and the like and achieves synergistic effects, particularly the antioxidant effect. Thus, the Cordyceps sinensis fermentation composition wholly achieves more excellent technical effects.

Therefore, in another aspect of the present disclosure, the provided Cordyceps sinensis fermentation composition has the EGT content of more than 0.004% (weight percentage), preferably more than 0.006% (weight percentage), preferably more than 0.008% (weight percentage), preferably more than 0.01% (weight percentage). The fermentation composition further includes one or more of adenosine, mannitol, amino acids, polysaccharides and erythritol.

Further, the Cordyceps sinensis fermentation composition has the erythritol content of more than 1% (weight percentage), preferably 1-6% (weight percentage), further preferably 1.5-5% (weight percentage), 1.5-4% (weight percentage), further preferably 2-5% (weight percentage), and further preferably 2.5-4% (weight percentage).

Further, the Cordyceps sinensis fermentation composition has the mannitol content of more than 4% (weight percentage), preferably more than 5%, preferably more than 6%, and preferably more than 7%.

More specifically, on the basis of the EGT content of more than 0.01%, the Cordyceps sinensis fermentation composition in the present disclosure has the erythritol content of more than 1% (weight percentage); or,

more specifically, on the basis of the EGT content of more than 0.01%, the Cordyceps sinensis fermentation composition in the present disclosure has the erythritol content of more than 1% (weight percentage) and the adenosine content of more than 0.02% (weight percentage); or,

more specifically, on the basis of the EGT content of more than 0.01%, the Cordyceps sinensis fermentation composition in the present disclosure has the erythritol content of more than 1% (weight percentage), the adenosine content of more than 0.02% (weight percentage) and the mannitol content of more than 5% (weight percentage); or,

more specifically, on the basis of the EGT content of more than 0.01%, the Cordyceps sinensis fermentation composition in the present disclosure has the adenosine content of more than 0.02% (weight percentage) and the mannitol content of more than 5% (weight percentage); or,

more specifically, on the basis of the EGT content of more than 0.01%, the Cordyceps sinensis fermentation composition in the present disclosure has the adenosine content of more than 0.02% (weight percentage) and the mannitol content of more than 6% (weight percentage); or,

more specifically, on the basis of the EGT content of more than 0.01%, the Cordyceps sinensis fermentation composition in the present disclosure has the adenosine content of more than 0.02% (weight percentage) and the mannitol content of more than 7% (weight percentage).

In another aspect of the present disclosure, the provided Cordyceps sinensis fermentation composition has the EGT content of more than 0.004% (weight percentage), preferably more than 0.006% (weight percentage), preferably more than 0.008% (weight percentage), preferably more than 0.01% (weight percentage), preferably the polysaccharide content of more than 2% (weight percentage); or,

further, the Cordyceps sinensis fermentation composition has the the polysaccharide content of more than 2% (weight percentage), preferably more than 3% (weight percentage), preferably 2-10% (weight percentage).

Further, on the basis of the EGT content of more than 0.01%, the Cordyceps sinensis fermentation composition in the present disclosure preferably has the the polysaccharide content of more than 2% (weight percentage) and total amino acid content of more than 20%.

More specifically, on the basis of the EGT content of more than 0.01%, the Cordyceps sinensis fermentation composition in the present disclosure preferably has the the polysaccharide content of 2-10% (weight percentage) and total amino acid content of 25-50%.

Those skilled in the art may understand that the above illustrations are merely for explaining the present disclosure, rather than limiting the present disclosure. With respect to the study on the EGT and adenosine content, the mannitol, the polysaccharides and the erythritol, the fermentation composition obtained in the scope has excellent technical effects.

In another aspect of the present disclosure, the provided Cordyceps sinensis fermentation composition has the EGT content of more than 0.004% (weight percentage), preferably more than 0.006% (weight percentage), preferably more than 0.008% (weight percentage), preferably more than 0.01% (weight percentage), and the erythritol content of more than 1% (weight percentage), preferably 1-6% (weight percentage), and further preferably 2-5% (weight percentage). When the content of the EGT and the erythritol contained in the Cordyceps sinensis fermentation composition provided by the present disclosure is within the preferred limited range of the present disclosure, the combination of the EGT and the erythritol achieves the synergistic effect on the hydroxyl radical scavenging rate, thereby further enhancing the overall oxidation resistance of the Cordyceps sinensis fermentation composition.

In another aspect of the present disclosure, the provided Cordyceps sinensis fermentation composition has the EGT content of more than 0.004% (weight percentage), preferably more than 0.006% (weight percentage), preferably more than 0.008% (weight percentage), preferably more than 0.01% (weight percentage), the adenosine content of more than 0.02% (weight percentage) and the mannitol content of more than 4% (weight percentage).

Further, the Cordyceps sinensis fermentation composition in the present disclosure has the EGT content of more than 0.01% (weight percentage), the adenosine content of more than 0.02% (weight percentage), the mannitol content of more than 4% (weight percentage) and the polysaccharide content of more than 2% (weight percentage).

Further, the Cordyceps sinensis fermentation composition in the present disclosure has the EGT content of more than 0.01% (weight percentage), the adenosine content of more than 0.02% (weight percentage), the mannitol content of more than 4% (weight percentage), and the total amino acid content of more than 20% (weight percentage), preferably 25-50% (weight percentage).

Further, the Cordyceps sinensis fermentation composition in the present disclosure has the EGT content of more than 0.01% (weight percentage), the adenosine content of more than 0.02% (weight percentage), the mannitol content of more than 4% (weight percentage), the polysaccharide content of more than 2% (weight percentage), and the total amino acid content of more than 20% (weight percentage), preferably 25-50% (weight percentage).

More specifically, according to the Cordyceps sinensis fermentation composition in the present disclosure, on the basis of the EGT content of more than 0.01%, preferably the adenosine content is more than 0.02% (weight percentage), the ratio of the EGT content to the adenosine content is 1:1-1:20, and the mannitol content is more than 5% (weight percentage); or,

more specifically, according to the Cordyceps sinensis fermentation composition in the present disclosure, on the basis of the EGT content of more than 0.01%, preferably the adenosine content is more than 0.03% (weight percentage), the ratio of the EGT content to the adenosine content is 1:1-1:20, and the mannitol content is more than 5% (weight percentage); or,

more specifically, according to the Cordyceps sinensis fermentation composition in the present disclosure, on the basis of the EGT content of more than 0.01%, preferably the adenosine content is more than 0.02% (weight percentage), the ratio of the EGT content to the adenosine content is 1:1-1:20, the mannitol content is more than 5% (weight percentage), and the polysaccharide content is more than 2% (weight percentage); or,

more specifically, according to the Cordyceps sinensis fermentation composition in the present disclosure, on the basis of the EGT content of more than 0.01%, preferably the adenosine content is more than 0.02% (weight percentage), the ratio of the EGT content to the adenosine content is 1:1-1:20, the mannitol content is more than 5% (weight percentage), and the total amino acid content is more than 20% (weight percentage); or,

more specifically, according to the Cordyceps sinensis fermentation composition in the present disclosure, on the basis of the EGT content of more than 0.01%, preferably the adenosine content is 0.02-1.0% (weight percentage), the ratio of the EGT content to the adenosine content is 1:1-1:20, and the mannitol content is more than 5% (weight percentage); or,

more specifically, according to the Cordyceps sinensis fermentation composition in the present disclosure, on the basis of the EGT content of more than 0.01%, preferably the adenosine content is 0.02-1.0% (weight percentage), the ratio of the EGT content to the adenosine content is 1:1-1:20, the mannitol content is more than 5% (weight percentage), and the polysaccharide content is more than 2% (weight percentage); or,

more specifically, according to the Cordyceps sinensis fermentation composition in the present disclosure, on the basis of the EGT content of more than 0.01%, preferably the adenosine content is 0.02-1.0% (weight percentage), the ratio of the EGT content to the adenosine content is 1:1-1:20, the mannitol content is more than 5% (weight percentage), and the total amino acid content is more than 20% (weight percentage); or,

more specifically, according to the Cordyceps sinensis fermentation composition in the present disclosure, on the basis of the EGT content of more than 0.01%, preferably the adenosine content is 0.02-1.0% (weight percentage), the ratio of the EGT content to the adenosine content is 1:1-1:20, the mannitol content is more than 5% (weight percentage), the polysaccharide content is more than 2% (weight percentage), and the total amino acid content is more than 20% (weight percentage).

Another aspect of the present disclosure further provides the Cordyceps sinensis fermentation composition including adenosine. The fermentation composition further includes EGT, and the EGT content (weight percentage) is more than 0.01%.

Further, the Cordyceps sinensis fermentation composition includes adenosine and mannitol. The fermentation composition further includes EGT, and the EGT content (weight percentage) is more than 0.01%.

Further, the Cordyceps sinensis fermentation composition includes adenosine, mannitol and a polysaccharide. The fermentation composition further includes EGT, and the EGT content (weight percentage) is more than 0.01%.

Further, the Cordyceps sinensis fermentation composition includes adenosine, mannitol, a polysaccharide and amino acids. The fermentation composition further includes EGT, and the EGT content (weight percentage) is more than 0.01%.

Further, the Cordyceps sinensis fermentation composition includes adenosine. The fermentation composition further includes EGT, the EGT content (weight percentage) is more than 0.01%, and the adenosine content is 0.02-1.0% (weight percentage).

Further, the Cordyceps sinensis fermentation composition includes adenosine. The fermentation composition further includes EGT, the EGT content (weight percentage) is more than 0.01%, and a ratio of the EGT content to the adenosine content is 1:1-1:20.

Further, the Cordyceps sinensis fermentation composition includes adenosine. The fermentation composition further includes EGT, the EGT content (weight percentage) is more than 0.01%, the adenosine content is 0.02-1.0% (weight percentage), and a ratio of the EGT content to the adenosine content is 1:1-1:20.

Further, the Cordyceps sinensis fermentation composition includes adenosine and mannitol. The fermentation composition further includes EGT, the EGT content (weight percentage) is more than 0.01%, the adenosine content is 0.02-1.0% (weight percentage), and the mannitol content is more than 4% (weight percentage).

Further, the Cordyceps sinensis fermentation composition includes adenosine and mannitol. The fermentation composition further includes EGT, the EGT content (weight percentage) is more than 0.01%, the adenosine content is 0.02-1.0% (weight percentage), a ratio of the EGT content to the adenosine content is 1:1-1:20, and the mannitol content is more than 4% (weight percentage).

Therefore, another aspect of the present disclosure further provides a Cordyceps sinensis fermentation composition. The EGT content is more than 0.004% (weight percentage), preferably more than 0.006% (weight percentage), preferably more than 0.008% (weight percentage), preferably more than 0.01% (weight percentage), the adenosine content is more than 0.02% (weight percentage), and the erythritol content is more than 1.0% (weight percentage).

More specifically, according to the Cordyceps sinensis fermentation composition in the present disclosure, on the basis of the EGT content of more than 0.01%, preferably the adenosine content is more than 0.02% (weight percentage), the ratio of the EGT content to the adenosine content is 1:1-1:20, and erythritol content is more than 1.0% (weight percentage); or,

more specifically, according to the Cordyceps sinensis fermentation composition in the present disclosure, on the basis of the EGT content of more than 0.01%, preferably the adenosine content is 0.02-1.0% (weight percentage), and the erythritol content is more than 1.0% (weight percentage).

Another aspect of the present disclosure further provides a Cordyceps sinensis fermentation composition including adenosine. The fermentation composition further includes EGT and erythritol, and the EGT content (weight percentage) is more than 0.01%.

Further, the Cordyceps sinensis fermentation composition includes adenosine and mannitol. The fermentation composition further includes EGT and erythritol, and the EGT content (weight percentage) is more than 0.01%.

Further, the Cordyceps sinensis fermentation composition includes adenosine, mannitol and a polysaccharide. The fermentation composition further includes EGT and erythritol, and the EGT content (weight percentage) is more than 0.01%.

Further, the Cordyceps sinensis fermentation composition includes adenosine, mannitol, a polysaccharide and amino acids. The fermentation composition further includes EGT and erythritol, and the EGT content (weight percentage) is more than 0.01%.

Further, the Cordyceps sinensis fermentation composition includes adenosine. The fermentation composition further includes EGT and erythritol, the EGT content (weight percentage) is more than 0.01%, and the adenosine content is 0.02-1.0% (weight percentage).

Further, the Cordyceps sinensis fermentation composition includes adenosine. The fermentation composition further includes EGT and erythritol, the EGT content (weight percentage) is more than 0.01%, and a ratio of the the EGT content to the adenosine content is 1:1-1:20.

Further, the Cordyceps sinensis fermentation composition includes adenosine. The fermentation composition further includes EGT and erythritol, the EGT content (weight percentage) is more than 0.01%, the adenosine content is 0.02-1.0% (weight percentage), and a ratio of the the EGT content to the adenosine content is 1:1-1:20.

Further, the Cordyceps sinensis fermentation composition includes adenosine and mannitol. The fermentation composition further includes EGT and erythritol, the EGT content (weight percentage) is more than 0.01%, the adenosine content is 0.02-1.0% (weight percentage), and the mannitol content is more than 4% (weight percentage).

Further, the Cordyceps sinensis fermentation composition includes adenosine and mannitol. The fermentation composition further includes EGT and erythritol, the EGT content (weight percentage) is more than 0.01%, the adenosine content is 0.02-1.0% (weight percentage), a ratio of the the EGT content to the adenosine content is 1:1-1:20, and the mannitol content is more than 4% (weight percentage).

Another aspect of the present disclosure further provides a Cordyceps sinensis fermentation composition including adenosine. The fermentation composition further includes EGT and erythritol, and the erythritol content is more than 1% (weight percentage).

Further, the Cordyceps sinensis fermentation composition includes adenosine and mannitol. The fermentation composition further includes EGT and erythritol, and the erythritol content is more than 1% (weight percentage).

Further, the Cordyceps sinensis fermentation composition includes adenosine, mannitol and a polysaccharide. The fermentation composition further includes EGT and erythritol, and the erythritol content is more than 1% (weight percentage).

Further, the Cordyceps sinensis fermentation composition includes adenosine, mannitol, a polysaccharide and amino acids. The fermentation composition further includes EGT and erythritol, and the erythritol content is more than 1% (weight percentage).

Further, the Cordyceps sinensis fermentation composition includes adenosine. The fermentation composition further includes EGT and erythritol, the erythritol content is more than 1% (weight percentage), and the adenosine content is 0.02-1.0% (weight percentage).

Further, the Cordyceps sinensis fermentation composition includes adenosine. The fermentation composition further includes EGT and erythritol, the EGT content is more than 0.01% (weight percentage), and the erythritol content is more than 1% (weight percentage).

Further, the Cordyceps sinensis fermentation composition includes adenosine. The fermentation composition further includes EGT and erythritol, the EGT content is more than 0.01% (weight percentage), the erythritol content is more than 1% (weight percentage), and the adenosine content is 0.02-1.0% (weight percentage).

Further, the Cordyceps sinensis fermentation composition includes adenosine and mannitol. The fermentation composition further includes EGT and erythritol, the EGT content (weight percentage) is more than 0.01%, the erythritol content is more than 1% (weight percentage), the adenosine content is 0.02-1.0% (weight percentage), and the mannitol content is more than 4% (weight percentage).

Another aspect of the present disclosure further provides a Cordyceps sinensis fermentation composition. The Cordyceps sinensis fermentation composition includes erythritol and mannitol, and the mannitol content is more than 1% (weight percentage), preferably 1-6% (weight percentage), further preferably 1.5-5% (weight percentage), 1.5-4% (weight percentage), further preferably 2-5% (weight percentage), more further preferably 2.5-4% (weight percentage). The Cordyceps sinensis. fermentation composition has the mannitol content of more than 4% (weight percentage), preferably more than 5%, preferably more than 6%, preferably more than 7%.

Further, the Cordyceps sinensis fermentation composition has polysaccharide content of more than 2% (weight percentage), preferably more than 3% (weight percentage), preferably 2-10% (weight percentage).

Further, the Cordyceps sinensis fermentation composition has adenosine content of more than 0.02% (weight percentage), more than 0.03% (weight percentage), preferably 0.03-1.00% (weight percentage), preferably 0.03-0.80% (weight percentage), preferably 0.03-0.60% (weight percentage), preferably 0.03-0.40% (weight percentage), preferably 0.03-0.20% (weight percentage), preferably 0.03-0.10% (weight percentage), preferably 0.03-0.08% (weight percentage).

Further, the Cordyceps sinensis fermentation composition has polysaccharide content of more than 2% (weight percentage), preferably more than 3% (weight percentage), preferably more than 4% (weight percentage), preferably 2-10% (weight percentage).

Further, the total amino acid content is more than 20% (weight percentage), preferably 25-50% (weight percentage).

The Cordyceps sinensis fermentation composition of the present disclosure can be directly prepared into various dosage forms, including powders, granules, tablets, pills, capsules, or by mixing the above components and packaging a mixture into capsules or tableting the mixture to obtain individual preparations. It can also be prepared into various dosage forms using acceptable carriers. With regard to orally administered compositions (for example, tablets and capsules), the term "acceptable carrier" includes such carriers as a conventional excipient, for example, an adhesive, such as syrup, acacia, gelatin, sorbitol, tragacanth, polyvinylpyrrolidone (povidone), methylcellulose, ethylcellulose, sodium carboxymethyl cellulose, hydroxypropyl methylcellulose, sucrose, and starch; a filler and a carrier such as corn starch, the gelatin, lactose, the sucrose, and microcrystalline cellulose; and a lubricant such as magnesium stearate, sodium stearate and other stearates, glyceryl monostearate, stearic acid, silicone fluid, talc, wax, oil, and colloidal silicon dioxide. A flavoring agent may also be used, for example, mint, wintergreen oil, and a cherry flavor. It may be necessary to add a coloring agent, in order to distinguish between dosage forms. The tablet may also be coated by a method well known in the art. The tablet may be compressed or molded, optionally including one or more auxiliary components. A compressed tablet may be such prepared by compressing a free-flowing form of the active components, such as the powders or the granules, in a suitable machine, optionally, mixed with one or more adhesives, lubricants, inert diluents, preservatives, surfactants, or dispersants.

In conclusion, the Cordyceps sinensis fermentation composition in the present disclosure is the first Cordyceps sinensis fermentation product having the EGT. Moreover, due to the study on the EGT and adenosine content, the fermentation composition obtained in the scope has an excellent antioxidant effect. Thus, the Cordyceps sinensis fermentation composition in the present disclosure wholly achieves more excellent technical effects compared with the prior art.

Therefore, the present disclosure further provides a pharmaceutical composition including a combination of EGT and adenosine. A combination ratio of the EGT to adenosine is 1:1-1:20.

Further, the ratio of the EGT content to adenosine content is 1:1-1:15, preferably 1:1-1:10, preferably 1:1-1:8, preferably 1:1-1:7, preferably 1:1-1:6, preferably 1:1-1:5, preferably 1:1-1:4, preferably 1:1-1:3, preferably 1:1-1:2.

The present disclosure further provides a pharmaceutical composition including a combination of EGT and erythritol. A combination ratio of the EGT to erythritol is 1:1-1:600, preferably 1:1-1:500, preferably 1:1-1:400, preferably 1:1-1:300, preferably 1:1-1:200, preferably 1:1-1:100.

The drug fermentation composition of the present disclosure can be directly prepared into various dosage forms, including the powders, the granules, the tablets, the pills, the capsules, or by mixing the above components and packaging a mixture into the capsules or tableting the mixture to obtain individual preparations. It can also be prepared into various dosage forms using acceptable carriers.

The drug composition of the present disclosure has the excellent antioxidant effect and the synergistic technical effect.

**Brief Description of the Drawings**

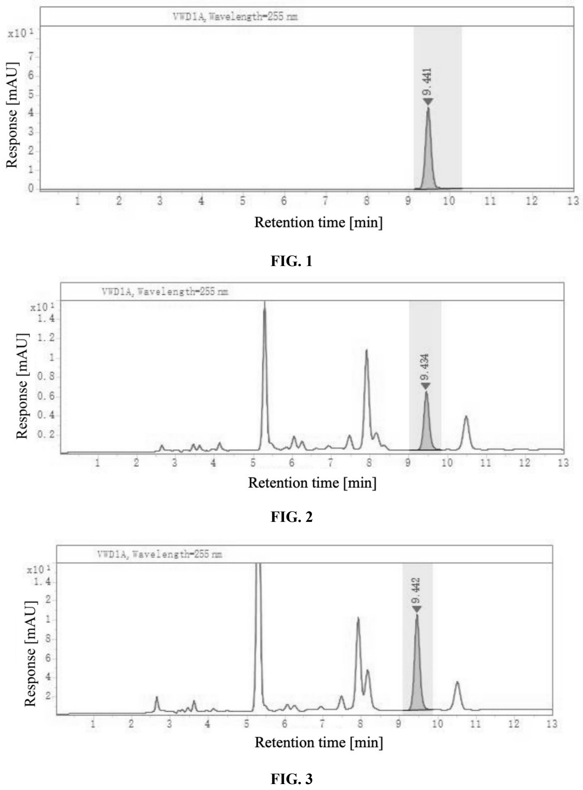

FIG. 1 is an HPLC spectrogram of an EGT standard substance;
FIG. 2 is an HPLC spectrogram of an EGT sample B-5;
FIG. 3 is an HPLC spectrogram of an EGT sample B-7;

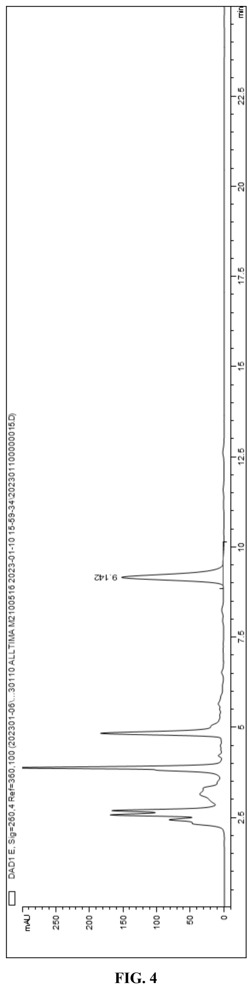

FIG. 4 is an HPLC spectrogram of a CN101096641A adenosine sample in embodiment 4;

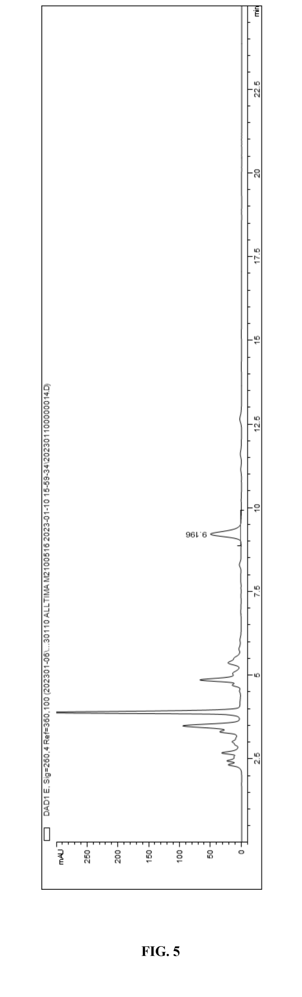

FIG. 5 is an HPLC spectrogram of an adenosine sample B-2 in embodiment 4;

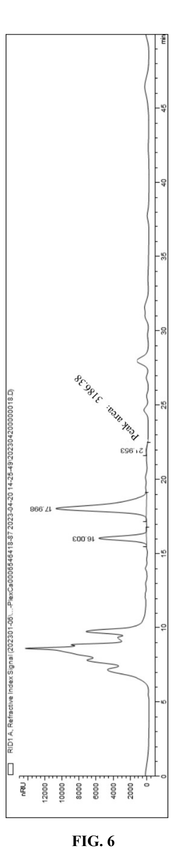

FIG. 6 is an HPLC spectrogram of an erythritol sample B-2;
FIG. 7 is a standard curve diagram of a polysaccharide.

**Detailed Description of the disclosure**

I. EGT detection method

The EGT is determined by high performance liquid chromatography (four parts 0512 of China Pharmacopoeia 2020 Edition).

Chromatographic condition and system applicability test

Octadecylsilane chemically bonded silica serves as a filling agent, 0.2% heptafluorobutyric acid serves as a mobile phase A, methanol serves as a mobile phase B, and a test wavelength is 255 nm. A number of theoretical plates should not be less than 300 according to an EGT peak.

Preparation of a reference solution

An appropriate amount of an EGT reference substance was precisely weighed, a 0.5% phosphoric acid solution was added to prepare a solution containing μg per mL, thereby obtaining the reference solution. The solution was subjected to gradient elution according to the following table:

| Time (min) | Mobile phase A(%) | Mobile phase B(%) |
|---|---|---|
| 0 | 100 | 0 |
| 12.5 | 100 | 0 |

(continued)

| Time (min) | Mobile phase A(%) | Mobile phase B(%) |
|---|---|---|
| 13 | 40 | 60 |
| 18 | 40 | 60 |
| 19 | 100 | 0 |
| 33 | 100 | 0 |

Preparation of a test solution

0.5 g of fermented Cordyceps sinensis mycelia powder was precisely weighed and added into a conical flask with cover; 20 mL of ether was added; the conical flask was capped; the solution was soaked for 30 minutes and filtered to remove the ether; the ether in the residue volatilized dry; the residue and filter paper were both placed in the conical flask with cover; 50 mL of 0.5% phosphoric acid solution was precisely added; the conical flask was capped; the solution was weighed and subjected to ultrasonic treatment for 30 minutes at the power of 250 W and a frequency of 33 kHz; the solution was cooled, shaken up and centrifuged; and the supernatant was taken, thereby obtaining the test solution.

Determination method

10 μL of the reference solution and 10 μL of the test solution were precisely absorbed respectively and injected into a liquid chromatograph, and the solution was determined.

II. Detection method of erythritol

Preparation of a reference solution

An appropriate amount of an erythritol reference substance was precisely weighed, deionized water was added into the erythritol reference substance to prepare a solution containing 1000 μg per mL, thereby obtaining the reference solution.

Preparation of a test solution

0.5 g of fermented Cordyceps sinensis mycelia powder was precisely weighed and added into a conical flask with cover; 10 mL of deionized water was precisely added; the conical flask was capped; the solution was weighed and subjected to ultrasonic treatment for 60 minutes (at the power of 250 W and a frequency of 40 kHz); the solution was cooled and then weighed; the lost weight was complemented with deionized water; the solution was shaken up and filtered; and the filtrate was taken, thereby obtaining the test solution.

Detection method

Detector: RID differential detector;
Chromatographic column: model of Agilent Hi-Plex Ca, 300×7.7 mm, 8 μm;
Mobile phase: deionized water;
Flow velocity of the chromatographic column: 0.6 mL/min; column temperature: 80°C; injection volume: 10 μL; detection time: 50 min;
Flow cell temperature: 35°C.

III. Anthrone-sulfuric acid detection method of polysaccharide

Solution preparation

Sulfuric acid-anthrone solution: 0.1 g of anthrone was precisely weighed; 100 mL of a sulfuric acid solution was added to dissolve the anthrone, and the solution was shaken up and placed in a brown bottle, thereby obtaining the sulfuric acid-anthrone solution.

Preparation of a standard curve

Preparation of a reference solution

An appropriate amount of an anhydrous dextrose reference substance was precisely weighed; water was added into the reference substance to prepare a solution including 0.1 mg per mL, thereby obtaining the reference solution.

Drawing of the standard curve

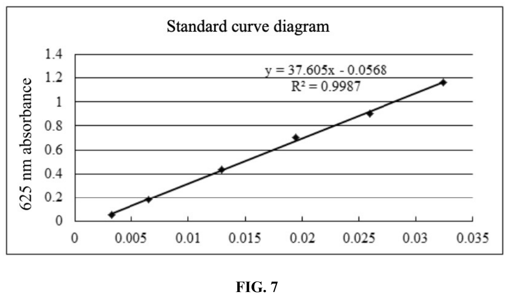

The reference solution was precisely measured by 0 mL, 0.2 mL, 0.4 mL, 0.8 mL, 1.2 mL, 1.6 mL and 2.0 mL respectively; each reference solution was placed in a 20 mL test tube with stopper; water was added into each test tube till the volume was up to 2.0 mL; 6 mL of the sulfuric acid-anthrone solution was rapidly precisely added; the solution was immediately shaken up and placed for 15 min; the test tubes were immediately placed in an ice-water bath for cooling for 15 min; the test tubes were taken out and placed at a room temperature for 10 min; a corresponding agent served as blank; absorbance was determined at 625 nm; and the standard curve was drawn by taking the absorbance as a vertical coordinate, as shown in Fig. 7.

Preparation of a test solution

2 g of fermented Cordyceps sinensis mycelia powder was precisely weighed and placed in a round-bottom flask; 60 mL of water was added into the flask for standing for 30 min; the solution was subjected to heating reflux for 3 h; the solution was transferred to a centrifuge tube when hot; the filter and the filter residue were washed with a small amount of hot water; the solution was centrifuged at 5000 rpm; the supernatant was taken; the precipitate was transferred to the round-bottom flask with a small amount of hot water; 60 mL of water was added; the solution was subjected to heating reflux for 2 h and transferred to a centrifuge tube when hot; the solution was centrifuged again; the supernatant twice was merged and then evaporated to dryness on a water bath; the residue was dissolved with 5 mL of water; 75 mL of ethanol was slowly added while stirring; the solution was shaken up, placed at 4°C for 12 h and then centrifuged; the supernatant was removed; the precipitate was dissolved with hot water and transferred to a 50 mL bottle; the solution was cooled; water was added to the scale; an appropriate amount of the solution was taken when shaken up and then centrifuged; 1 mL of the supernatant was precisely measured and placed in a 25 mL measuring flask; water was added to the scale; and the solution was shaken up, thereby obtaining the test solution.

Determination

2 mL of the test solution was precisely measured and placed in a 10 mL test tube with stopper; the same operation was conducted according to the method under the standard curve preparation item when "6 mL of the sulfuric acid-anthrone solution was rapidly and precisely added"; the absorbance was determined; content of the anhydrous dextrose in the test solution was read from the standard curve; and the result was obtained through calculation.

Calculation of the result

$$W = \frac{c \times 8/2 \times 25/2 \times 50}{m} \times 100$$

In the formula:

W represents the content of polysaccharide, %;
c represents the polysaccharide concentration of the sample detected on the standard curve, mg/mL;
m represents sample mass, mg;
8/2 and 25/2 represent dilution ratios;
50 represents a volume value of the precipitate with a fixed volume by hot-water dissolving after water extraction and alcohol precipitation.

IV. Detection of hydroxyl radical scavenging rate (Fenton colorimetric method)

Detection principle

$H_2O_2/Fe^{2+}$ produces a hydroxyl radical through a Fenton reaction, $Fe^{2+}$ is oxidized into $Fe^{3+}$, and red phenanthro-line-$Fe^{2+}$ is oxidized into colorless phenanthroline-$Fe^{3+}$, so that the maximum absorption peak of the phenanthroline-$Fe^{2+}$ disappears at 536 nm. The absorbance change at 530-540 nm may be determined by a spectrophotometer. Thus, content change of the hydroxyl radical can be calculated, that is, the hydroxyl radical scavenging rate or scavenging capacity of the sample can be calculated.

Solution preparation

1. 0.2 g of mycelia powder was weighed; 2 mL of purified water was added; the mycelia powder and the water were uniformly mixed; the mixture was placed at a room temperature for standing for 4 h; the solution was centrifuged at 10000 rpm for 10 min; and the supernatant was taken for later use.

2. Other samples served as reference solutions.

The spectrophotometer was started for preheating for 30 min; the wavelength was adjusted to 536 nm; and the purified water was subjected to zero setting.

Sample injection

A blank tube, an undamaged tube, a damaged tube, a control tube and a determination tube were set according to the following table; the solutions were added in sequence; and the solutions were uniformly mixed and placed in a water bath at 37°C for 1 h.

| Added substances (mL) | Blank tube A0 | Undamage d tube A1 | Damage d tube A2 | Control tube A3' | Determi nation tube A3 |
|---|---|---|---|---|---|
| Phenanthroline solution | - | 0.6 | 0.6 | - | 0.6 |
| OH Assay buffer | 0.8 | 0.8 | 0.8 | 0.8 | 0.8 |
| Ferrous developing solution | - | 0.4 | 0.4 | - | 0.4 |
| Purified water | 3.2 | 2.2 | 1.8 | 2.8 | 1.4 |
| To-be-tested solution | - | - | - | 0.4 | 0.4 |
| Oxidizing agent | - | - | 0.4 | - | 0.4 |

To-be-tested sample•OH scavenging rate (%) = [(A3 - A3') - (A2 - A0)]/(A1 - A2) $\times$ 100%        Calculation:

V. Detection methods of adenosine, mannitol, total amino acid content and water were conducted according to the detection method under the fermented Cordyceps sinensis. mycelia powder item in accordance with National Drug Standards.

VI. Detection method of tyrosinase inhibition rate

1. Preparation of a solution:

(1) Preparation of a PBS: 3.4022 g of potassium dihydrogen phosphate and 5.7055 g of potassium phosphate dibasic trihydrate were respectively weighed and then dissolved with an appropriate amount of distilled water; the volume was fixed in a 1000 mL volumetric flask; the solution was shaken up to obtain the PBS having a pH value of 6.81 and a concentration of 0.05 mol·L-1, and the PBS was kept in a dark place at 4°C in a refrigerator.
(2) Preparation of an L-Dopa solution: 0.0395 g of L-Dopa was precisely weighed and dissolved with the prepared PBS; the solution was transferred into a 100 mL volumetric flask; the volume was fixed; the solution was shaken up and subjected to ultrasonic treatment to obtain the L-Dopa solution of 2 mmol·L-1. The solution was used when prepared.

(3) Preparation of a TYR (tyrosine kinase) solution: 25 KU of the TYR was totally dissolved with a small amount of PBS; the solution was repeatedly washed for several times; the washing liquid was transferred into the 1000 mL volumetric flask; the volume was fixed with the PBS; the liquid was shaken up to obtain a TYR solution of 250 U·mL-1; then the mother solution was diluted to 125 U·mL-1; and the solution was preserved at -20°C in the refrigerator for later use.

2. Preparation of samples: 250 mg/ml of an erythritol mother solution: 2.5 g of erythritol was weighed, and 10 ml of purified water was added; 250 mg/ml of a mannitol mother solution: 7.5 g of mannitol was weighed, 30 ml of purified water was added, the mixture was heated and dissolved; and the solution was diluted into the needed concentration according to each reaction system and then added into the detection system.

3. Determination of the sample on TYR inhibitory activity

The reaction system was shown as Table 4.2. 2 mmol·L-1 of L-Dopa served as a substrate; the PBS, the sample solution and the substrate were added into 96-well plates numbered as 1, 2, 3 and 4 in sequence; the operation was repeated for three times for each well; the temperature of a microplate reader was set as 37°C; the plates were linearly shaken for 10 min and then taken out; finally the TYR solution was added; the plates were rapidly put back; a detection wavelength was 475 nm; a total volume of the reaction system was 240 μL; the absorbance was measured once every 30 s; total recording time was 30 min; and an inhibition rate was calculated based on the absorbance within 30 min. A calculation formula of the inhibition rate was as follows: when the calculated result was negative, the absolute value of the negative was an activation rate.

| Composition of the reaction solution | Volume of the reaction solution/μL | | | |
|---|---|---|---|---|
| | A1 | A2 | A3 | A4 |
| PBS buffer | 80 | 160 | 0 | 80 |
| Sample solution | 0 | 0 | 80 | 80 |
| Substrate | 80 | 80 | 80 | 80 |
| TYR | 80 | 0 | 80 | 0 |

$$\text{Inhibition rate} = \frac{(A_1 - A_2) - (A_3 - A_4)}{A_1 - A_2} \times 100\% \qquad (4.1)$$

A1: absorbance of a reaction solution with TYR instead of the sample determined at 475 nm;
A2: absorbance of a reaction solution without the sample nor the TYR determined at 475 nm;
A3: absorbance of a reaction solution with both the sample and the TYR determined at 475 nm;
A4: absorbance of a reaction solution with the sample instead of the TYR determined at 475 nm.

**Embodiment 1 Content of EGT and erythritol in different mycelia**

Researches of the inventor show that, the EGT is not detected from culture soil, larva and silkworm pupa of natural Cordyceps sinensis, and only 0.008% of EGT is detected from partial fresh larva and dried larva. Therefore, the EGT is difficultly detected from the mycelia of the natural Cordyceps sinensis.

| Sample | EGT content mg/g | EGT content % | Erythritol content % |
|---|---|---|---|
| Fresh larva | 0.06 | 0.006% | Not detected |
| Dried larva | 0.08 | 0.008% | Not detected |
| Muscardine cadaver (dried) | 0.08 | 0.008% | Not detected |
| Larva | Not detected | Not detected | Not detected |
| Culture soil | Not detected | Not detected | Not detected |

Although theoretically the content and medicinal effects of nutrient substances in the mycelia are similar to those of natural Cordyceps sinensis by virtue of fermentation of fungi separated from the natural Cordyceps sinensis, there are still differences between components of the Cordyceps sinensis fermentation product and the components of the natural Cordyceps sinensis, particularly metabolic products and secondary metabolic products in the fermentation process have differences from the natural Cordyceps sinensis and also have significant differences from different Cordyceps sinensis fermentation products. On the basis of researching the natural Cordyceps sinensis, Hirsutella sinensis strains collected in the collection center, such as Hirsutella sinensis in China Center for Type Culture Collection (CCTCC) having a collection number of CCTCC No:M 2011278, Hirsutella sinensis CGMCC 3.14240 and Hirsutella sinensis CGMCC3.14243 purchased from China General Microbiological Culture Collection Center (CGMCC), and Paecilomyces hepiali 3.15540, Paecilomyces hepiali CGMCC3.7845 and Paecilomyces hepiali CGMCC3.7908 purchased from the CGMCC were researched. Further, with reference to the prior art, on the basis of eliminating possible interference of ectogenic EGT (for example, the EGT is not detected from silkworm pupa powder serving as a culture medium), it is researched that, content of the EGT in fermentation filtrate and dried mycelium powder in the current Cordyceps sinensis fermentation process is lower than the amount of detectable EGT in the natural Cordyceps sinensis, and the EGT is not detected in most of the fermentation filtrate and dried mycelium powder. However, it is accidentally discovered by the inventor that, tiny EGT can be obtained from the fermented mycelia of the Hirsutella sinensis.

| Strains | Fermented with reference to the prior art | EGT content % | |
| --- | --- | --- | --- |
| | | Fermentati on filtrate | Dried mycelium powder |
| Hirsutella sinensis, having a collection number of CCTCC No:M 2011278 | CN101096641A embodiment 1 CN108384726A embodiment 5 CN102373190A embodiment 1 | Not detected Not detected Not detected | 干 干 干 |
| Hirsutella sinensis CGMCC 3.14240 | | Not detected Not detected Not detected | 干 干 干 |
| Hirsutella sinensis CGMCC3.14243 | | Not detected Not detected Not detected | 干 干 干 |
| Paecilomyces hepiali CGMCC3.15540 | CN108553487A embodiments 1-2 | Not detected | Not detected |
| Paecilomyces hepiali CGMCC3.7845 | | Not detected | Not detected |
| Paecilomyces hepiali CGMCC3.7908 | | Not detected | Not detected |
| Notes: 干 represents: the content is 0.3-1.0% that of the natural Cordyceps sinensis only according to the peak area. | | | |

As shown in the above table, fermentation is conducted according to the method disclosed in the prior art, and significant EGT is not obtained from the fermentation filtrate and the mycelia in the fermentation process.

Further, in addition to the available strains in the collection center, commercially available Hirsutella sinensis strains, such as Ophiocordyceps sinensis (Hirsutella hepiali, Hirsutella sinensis) standard strains CICC 14092, CICC 14095, CICC 14097 and CICC 14099 purchased from China Center of Industrial Culture Collection (CICC), Ophiocordyceps sinensis (Hirsutella hepiali, Hirsutella sinensis) strains having article numbers of TS326189 and TS326199 purchased from TESTO Biology, and Hirsutella sinensis strains having article numbers of TS361737 and TS392986, are fermented with reference to the prior art, while the EGT is not obtained from the obtained fermentation filtrate and mycelia.

Similarly, commercially available Paecilomyces hepiali strains, such as strains having collection numbers of CGMCC 3.15421, CGMCC 3.14870, CGMCC 3.12108, CGMCC 3.10157, CCTCC NF 20081231, CCTCC NF 20081239, CCTCC NF 20081244 and CCTCC NF 20081250 are fermented by the inventor with reference to the prior art, while the EGT is not obtained from the obtained fermentation filtrate and mycelia.

| Strains | Fermented with reference to the prior art | Erythritol content % |
|---|---|---|
| | | Dried mycelium powder |
| Hirsutella sinensis, having a collection number of CCTCC No:M 2011278 | CN108384726A embodiment 5 | 0.8% |
| Hirsutella sinensis CGMCC 3.14240 | | |
| Hirsutella sinensis CGMCC3.14243 | | |
| Paecilomyces hepiali CGMCC3.15540 | CN108553487A embodiments 1-2 | 0.34% |
| Paecilomyces hepiali CGMCC3.7845 | | 0.3% |

As shown in the above table, fermentation is conducted by the inventor according to the method disclosed in the prior art, and stable and significant erythritol is not obtained from the fermentation filtrate and the mycelia in the fermentation process.

Further, in addition to the available strains in the collection center, commercially available Hirsutella sinensis strains, such as Ophiocordyceps sinensis (Hirsutella hepiali, Hirsutella sinensis) standard strains CICC 14092, CICC 14095, CICC 14097 and CICC 14099 purchased from China Center of Industrial Culture Collection (CICC), Ophiocordyceps sinensis (Hirsutella hepiali, Hirsutella sinensis) strains having article numbers of TS326189 and TS326199 purchased from TESTO Biology, and Hirsutella sinensis strains having article numbers of TS361737 and TS392986, are fermented with reference to the prior art, while stable and significant erythritol is not obtained from the obtained fermentation filtrate and mycelia.

Similarly, commercially available Paecilomyces hepiali strains, such as strains having collection numbers of CGMCC 3.15421, CGMCC 3.14870, CGMCC 3.12108, CGMCC 3.10157, CCTCC NF 20081231, CCTCC NF 20081239, CCTCC NF 20081244 and CCTCC NF 20081250 are fermented by the inventor with reference to the prior art, while the stable and significant erythritol is not obtained from the obtained fermentation filtrate and mycelia.

**Embodiment 2 Fermentation product having stable EGT content of more than 0.004%**

A Hirsutella sinensis strain having a collection number of CCTCC No: M 2011278 obtained from the China Center for Type Culture Collection (CCTCC) was inoculated on a slant plane, and was subjected to slant culture, shake-flask/seed flask culture and fermentation culture.

1 Slant culture

1.1 Formula of a slant solid medium: 1.8% of silkworm pupa powder, 1.5% of corn meal, 2.0% of glucose, 0.01% of $MgSO_4$, 0.02% of $KH_2PO_4$, 1.0-3.0 g/60 mL of oatmeal, 0.65% of agar powder, having a pH value of 6.8-7.2, prepared with water, and sterilized at (118-122)°C for (30±1) min.

1.2 Under an aseptic condition, about $2\times2$ cm mycelium block was dug away by an inoculating shovel, mycelia were uniformly coated on a blank solid medium; and the medium was placed in a biochemical incubator for culture at 15°C for 30 days.

1.3 The cultured slant plane was preserved at a low temperature of 0-8°C in a refrigerator, the mycelia grown slowly during the period, and the mycelia coated with the slant plane were taken out for later use.

2 Shake-flask culture

2.1 Formula of shake-flask culture medium: 2.0% of silkworm pupa powder, 2.5% of corn meal, 2.5% of bran, 0.25% of fish peptone, 0.75% of peptone, 0.02% of $KH_2PO_4$, 3.0% of glucose and 0.01% of $MgSO_4$, having a pH value of 6.8-7.2.

2.2 Liquidation: the silkworm pupa powder, corn meal and bran were weighed according to a preparation volume of a liquidation material; water was added for liquidation at (118-122)°C for 30 min; the liquidation material was taken out for standing till top-bottom layering; and the material was filtered by a filter screen with 6 layers of gauze so as to obtain the filtrate.

2.3 Preparation of the culture medium: the corresponding materials were weighed and dissolved with the liquefied filtrate, the volume was fixed, and the filtrate was filled into the shake flask with a volume of 1500mL/5000mL, and sterilized at (118-122)°C for (30±1) min.

2.4 Digging blocks: the slant plane was cut into about 1.5×2.0 cm mycelium blocks, and the cut mycelium blocks were raked into the prepared digging block/seed flask culture medium using a sterile rake, and subject to shaking culture for 9-14 days at (13-16)°C, (120-140)rpm.

3. Fermentation culture:

3.1 Basic fermentation medium formula: 2.8% glucose, 1% yeast extract, 0.02% $KH_2PO_4$, 0.01% $MgSO_4$, and pH 7.0.

3.2 Preparation of the culture medium: the filtrate in the shake flask has a volume of 1000mL/5000mL, and sterilized at (118-122)°C for (30±1) min.

3.3 Fermentation: the inoculation amount was 10%, and the filtrate is subject to shaking culture for 7 days at (13-16)°C, (120-140) rpm before bottling.

3.4 An appropriate volume of fermentation broth was measured and subject to vacuum filter to obtain wet mycelium slices, and the wet mycelium slices were dried to obtain bacterial powder samples.

Different ingredients were added to the above basic fermentation medium (2.8% glucose, 1% yeast extract, 0.02% $KH_2PO_4$, and 0.01% $MgSO_4$ (% refers to a weight-to-volume ratio)), and the mycelium experiment number samples A-1 to A-8 and B-1 to B-8 were obtained by fermentation. The results of detecting the EGT and the erythritol are shown in the following table:

| Experiment No. | Nitrogen sources added based on the fermentation medium containing 2.8% glucose, 1% yeast extract, 0.02% $KH_2PO_4$, and 0.01% $MgSO_4$ | | | EGT content % | Adenosine content % | Mannitol content % | Erythritol content % | Dry weight of mycelia (g/L) |
|---|---|---|---|---|---|---|---|---|
| A-1 | **6mM methionine added** | Pea protein | **1** % | 0.009 | 0.038 | 5.8 | 1.86 | 13.35 |
| A-2 | | Wheat protein | **1** % | 0.008 | 0.042 | 7.4 | 2.66 | 11.33 |
| A-3 | | Potato protein | **1**% | 0.005 | 0.025 | 4.8 | 0.91 | 11.94 |
| A-4 | | Spud protein | **1**% | 0.006 | 0.026 | 4.9 | 0.99 | 13.12 |
| A-5 | | Corn protein powder | **1**% | 0.003 | 0.042 | 7.6 | 2.63 | 10.00 |
| A-6 | | Peptide powder 101 | **1** % | 0.005 | 0.028 | 5.6 | 1.47 | 10.64 |
| A-7 | | Peptide powder 102 | **1** % | 0.009 | 0.033 | 7.1 | 2.14 | 12.00 |
| A-8 | | **Peptide powder 607** | **1** % | **0.013** | **0.050** | **8.4** | **3.63** | **8.66** |

(continued)

| Experiment No. | Nitrogen sources added based on the fermentation medium containing 2.8% glucose, 1% yeast extract, 0.02% KH$_2$PO$_4$, and 0.01% MgSO$_4$ | | EGT content % | Adenosine content % | Mannitol content % | Erythritol content % | Dry weight of mycelia (g/L) |
|---|---|---|---|---|---|---|---|
| B-1 | **Methionine not added** | **Peptide powder 607** | **1 %** | **0.014** | **0.046** | **9.1** | 1.03 | **10.22** |
| B-2 | | Peptide powder 705 | 1 % | 0.023 | 0.049 | 9.3 | 1.83 | 8.96 |
| B-3 | | Peptide powder 308 | 1 % | 0.022 | 0.048 | 9.4 | 1.79 | 7.58 |
| B-4 | | Peptide powder 702 | 1% | 0.026 | 0.050 | 9.6 | 1.67 | 8.26 |
| B-5 | | Peptide powder 203 | 1% | 0.018 | 0.047 | 9.3 | 1.34 | 11.36 |
| B-6 | | Peptide powder 305 | 1% | 0.023 | 0.050 | 9.5 | 2.33 | 7.99 |
| B-7 | | 0.5% peptide powder 203 + 0.5% fish peptone | 1% | 0.031 | 0.052 | 9.7 | 1.03 | 10.41 |

Further, in addition to the strains disclosed in the above patents, commercially available Hirsutella sinensis strains, such as Hirsutella sinensis CGMCC 3.14240 and Hirsutella sinensis CGMCC3.14243 purchased from the China General Microbiological Culture Collection Center (CGMCC), Ophiocordyceps sinensis (Hirsutella hepiali, Hirsutella sinensis) standard strains CICC 14092, CICC 14095, CICC 14097 and CICC 14099 purchased from China Center of Industrial Culture Collection (CICC), Ophiocordyceps sinensis (Hirsutella hepiali, Hirsutella sinensis) strains having article numbers of TS326189 and TS326199 purchased from TESTO Biology, and Hirsutella sinensis strains having article numbers of TS361737 and TS392986, are fermented through repeated experiments according to the fermentation method of Hirsutella sinensis having the preservation number of CCTCC No: M 2011278 in Embodiment 2 under the conditions of different culture media shown in the above table. The EGT content, adenosine content%, and mannitol and erythritol content% in the bacterial powder samples obtained by fermentation of different Hirsutella sinensis are not significantly different from the contents of the components in the bacterial powder prepared with Hirsutella sinensis having the preservation number of CCTCC No: M 2011278 under the same conditions.

**Embodiment 3 Study on the effect of effective amount of EGT or erythritol on the efficacy of Hirsutella sinensis fermented bacterial powder**

1. Experimental results of synergistic effect of EGT and adenosine on hydroxyl radical scavenging rate

| Experimental samples | OH clearance rate (%) |
|---|---|
| EGT 0.4 ml (0.02 mg/ml) | -5.70% |
| Adenosine 0.4 ml (0.02 mg/ml) | 0.00% |
| EGT 0.2 ml (0.02 mg/ml) + Adenosine 0.2 ml (0.02 mg/ml) | 3.03% |

2. Experimental results of synergistic effect of EGT and erythritol on hydroxyl radical scavenging rate

| Experimental samples | OH clearance rate (%) |
|---|---|
| EGT 0.4 ml (0.05 mg/ml) | 2.08% |
| Erythritol 0.4 ml (0.05 mg/ml) | -0. 05% |
| EGT 0.2 ml (0.05 mg/ml) + Erythritol 0.2 ml | 3.39% |
| (0.05 mg/ml) | |

3. Experimental results of synergistic effect of erythritol and adenosine on hydroxyl radical scavenging rate

| Experimental samples | OH clearance rate (%) | Theor etical values |
|---|---|---|
| Erythritol 12.5 mg (6.25 mg/ml) | -8.17% | - |
| Adenosine 0.15 ml (0.75 mg/ml) | 10.57% | - |
| Erythritol 12.5 mg (6.25 mg/ml) + Adenosine 0.15 ml (0.75 mg/ml) | 13.83% | 2.4% |

| Experimental samples | OH clearance rate (%) | Theor etical values |
|---|---|---|
| Erythritol 12.5 mg (6.25 mg/ml) | -5.8% | - |
| Adenosine 0.15 ml (2.5 mg/ml) | 7.17% | - |
| Erythritol 12.5 mg (6.25 mg/ml) + Adenosine 0.15 ml (2.5 mg/ml) | 9.06% | 1.37% |

The theoretical values stated in the table are the simple superposition of the hydroxyl radical scavenging rates of the two components;
From the above data, it can be seen that the composition of the erythritol and the adenosine produces a significant synergistic effect, and the hydroxyl free radical scavenging effect of the two components is at least 4.76 times higher than the theoretical value.

**4. Synergistic effect of erythritol and mannitol on tyrosinase inhibition rate**

| Experimental samples | Tyrosinase inhibition rate (%) |
|---|---|
| Erythritol 1.25 mg (6.25 mg/ml) | 1.71% |
| Mannitol 1.25 mg (6.25 mg/ml) | 2.51% |
| Erythritol 1.25 mg (6.25 mg/ml) + Mannitol 1.25 mg (6.25 mg/ml) | 10.62% |

| Experimental samples | Tyrosinase inhibition rate (%) |
|---|---|
| Erythritol 1.75 mg (8.75 mg/ml) | 6.39% |
| Mannitol 2.5 mg (12.5 mg/ml) | 2.51% |
| Erythritol 1.75 mg (8.75 mg/ml) + Mannitol 2.5 mg (12.5 mg/ml) | 10.62% |

| Experimental samples | Tyrosinase inhibition rate (%) |
|---|---|
| Erythritol 0.75 mg (3.75 mg/ml) | -1.06% |
| Mannitol 1.25 mg (6.25 mg/ml) | 3.62% |
| Erythritol 0.75 mg (3.75 mg/ml) + Mannitol 1.25 mg (6.25 mg/ml) | 5.21% |

5. Experimental results of synergistic effect of erythritol and mannitol on hydroxyl free radical scavenging rate

| Experimental samples | OH clearance rate (%) |
| --- | --- |
| Erythritol 1.5 mg (7.5 mg/ml) | 2.44% |
| Mannitol 2.5 mg (12.5 mg/ml) | 7.48% |
| Erythritol 1.5 mg (7.5 mg/ml) + Mannitol 2.5 mg (12.5 mg/ml) | 49.92% |
| Erythritol 1.75 mg (8.75 mg/ml) | 11.76% |
| Mannitol 2.5 mg (12.5 mg/ml) | 7.48% |
| Erythritol 1.75 mg (8.75 mg/ml) + Mannitol 2.5 mg (12.5 mg/ml) | 39.54% |
| Erythritol 2 mg (10 mg/ml) | 20.15% |
| Mannitol 2.5 mg (12.5 mg/ml) | 7.48% |
| Erythritol 2 mg (10 mg/ml) + Mannitol 2.5 mg (12.5 mg/ml) | 43.97% |
| Erythritol 2.5 mg (12.5 mg/ml) | 11.45% |
| Mannitol 2.5 mg (12.5 mg/ml) | 7.48% |
| Erythritol 2.5 mg (12.5 mg/ml) + Mannitol 2.5 mg (12.5 mg/ml) | 23.66% |
| Erythritol 0.75 mg (3.75 mg/ml) | 5.14% |
| Mannitol 2.5 mg (12.5 mg/ml) | 17.91% |
| Erythritol 0.75 mg (3.75 mg/ml) + Mannitol 2.5 mg (12.5 mg/ml) | 25.7% |

**Embodiment 4 Experimental results on hydroxyl radical scavenging rate of fermented bacterial powder obtained by the present disclosure and fermented bacterial powder in the prior art**

**Experimental samples:**

The preparation of mycelium samples B-2, B-5, and B-7 was the same as before;

Sample preparation in Embodiment 5 of CN108384726A:

2.0% of sorbitol, 0.1% of sea cucumber extract and 0.15% of Angelica sinensis extract were added to the basic culture medium described in Embodiment 1, and the mycelium was fermented in the culture medium.

Sample preparation in Embodiment 1 of CN101096641A:

The strains are inoculated on the slant plane. The culture medium formula consists of 1.5% of glucose, 1.0% of corn meal, 0.5% of dextrin, 1.5% of bran, 0.5% of yeast powder, 1.5% of silkworm pupa powder, 1.0% of peptone, 0.01% of magnesium sulfate, 0.02% of potassium dihydrogen phosphate, and 0.8% of agar powder, and the rest is water. The culture medium was cultured at 12°C for 25 days; and then the strains are inoculated in the fermentation medium. The culture medium formula consists of 1.0% of glucose, 1.0% of molasses, 0.5% of silkworm pupa powder, 1.0% of soybean cake meal, 0.5% of yeast extract, 0.01% of magnesium sulfate, and 0.02% of potassium dihydrogen phosphate, and the rest is water. The culture medium was placed on a shaker, and cultured at 14°C for 8 days; and then inoculated in the first-level fermentation tank, and cultured at 14°C for 8 days, expanded by 10 times the amount of the culture medium, and fermented step by step, until reaching a 30 t tank. Solid-liquid separation is carried out after pouring from the tank, and the solid is dried and crushed.

| | Experimen tal samples | EGT content % | Adenos ine content % | Erythrito l% | Manni tol conten t% | OH clearan ce rate (%) |
| --- | --- | --- | --- | --- | --- | --- |
| Embodime | B-2 | 0.023 | 0.049 | 1.83 | 9.3 | 55.89 |

(continued)

| | Experimen tal samples | EGT content % | Adenos ine content % | Erythrito 1% | Manni tol conten t% | OH clearan ce rate (%) |
|---|---|---|---|---|---|---|
| nts of the disclosure | B-5 | 0.018 | 0.047 | 1.34 | 9.3 | 56.97 |
| Control embodi-ments | CN1083847 26A | 干 | 0.20 | 0.18 | 8.2 | -5.07 |
| | CN1010966 41A | 干 | 0.24 | 0.64 | 8.0 | -5.21 |

Those skilled in the art would understand that the specific embodiments listed above are only illustrative of the present disclosure and are not construed as limiting the present disclosure. At the same time, those skilled in the art would understand that, in accordance with the spirit of the present disclosure, they can implement different preparation embodiments to achieve the same technical effects, which are all included in the scope of the present disclosure.

Industrial Applicability

The Cordyceps sinensis fermentation composition provided by the present disclosure is the first Cordyceps sinensisfermentation product containing the EGT, and due to the study on the content of the EGT and the adenosine, the fermentation composition obtained within the range has a very excellent antioxidant effect, so that the Cordyceps sinensis fermentation composition of the present disclosure shows a more excellent technical effect than the prior art as a whole, and thus has strong industrial applicability.

**Claims**

1.    A Cordyceps sinensis fermentation composition, **characterized by** comprising erythritol having a content of more than 1 wt%, preferably, 1-6 wt%, further preferably, 2-5 wt%, more further preferably, 2.5-4 wt%.

2.    The fermentation composition according to claim 1, **characterized in that** the fermentation composition further comprises adenosine having a content of more than 0.02 wt%, preferably, 0.03 wt%-1.0 wt%.

3.    The fermentation composition according to claim 1 or 2, **characterized in that** the fermentation composition further comprises ergothioneine having a content of more than 0.004 wt%, preferably, more than 0.01 wt%.

4.    The fermentation composition according to any one of claims 1-3, **characterized in that** the fermentation composition further comprises one or more of mannitol, an amino acid and polysaccharide.

5.    The fermentation composition according to claim 4, **characterized in that** a content of the mannitol is more than 5 wt%, preferably, more than 6 wt%, further preferably, more than 7 wt%.

6.    The fermentation composition according to claim 4 or 5, **characterized in that** a content of the polysaccharide is more than 2 wt%, preferably, more than 3 wt%, further preferably, more than 4 wt%, more further preferably, 2-10 wt%.

7.    The fermentation composition according to any one of claims 4-6, **characterized in that** a total content of the amino acid is more than 20 wt%, preferably, 25-50 wt%.

8.    The fermentation composition according to any one of claims 1-7, **characterized in that** the fermentation composition is prepared by fermentation using Hirsutella sinensis.

9.    The fermentation composition according to any one of claims 1-8, **characterized in that** the fermentation composition is used in preparation of drugs or health food products.

**FIG. 1**

**FIG. 2**

**FIG. 3**

**FIG. 4**

**FIG. 5**

**FIG. 6**

**FIG. 7**

## INTERNATIONAL SEARCH REPORT

| International application No. |
|---|
| **PCT/CN2024/099118** |

| A. | CLASSIFICATION OF SUBJECT MATTER |
|---|---|

A23L33/125(2016.01)i; A61K36/068(2006.01)i; A61P39/06(2006.01)i

According to International Patent Classification (IPC) or to both national classification and IPC

| B. | FIELDS SEARCHED |
|---|---|

Minimum documentation searched (classification system followed by classification symbols)

IPC:A23L,A61K,A61P

Documentation searched other than minimum documentation to the extent that such documents are included in the fields searched

Electronic data base consulted during the international search (name of data base and, where practicable, search terms used)

CJFD, CNTXT, DWPI, ENTXT, ENTXTC, OETXT, VEN, WPABSC, NCBI, ISI_Web of Science, 读秀, DUXIU, 百度学术, BAIDU SCHOLAR:杭州中美华东制药江东有限公司,杭州中美华东制药有限公司,中国远大集团有限责任公司,许峰,滕毅,张辉,张璇,陈志杰,姜辉,李雅佩,王鸿艳,王洁,虫草,冬虫夏草,中国被毛孢,中华被毛孢,蝙蝠蛾被毛孢,发酵,培养,赤藓醇,赤藓糖醇,丁四醇,腺苷,麦角硫因,麦硫因,硫组氨酸甲基内盐,麦角含硫碱,甘露醇,甘露糖醇,抗氧化,Cordyceps sinensis, Ophiocordyceps sinensis, Chinese caterpillar fungus, Hirsutella sinensis, Trichosporium hobmoth, Trichosporium chinensis, ferment+, cultivat+, Erythritol, Butanetetraol, Adenosine, Ergothioneine, Thiophistidinemethyl endosalt, Ergot sulfur base, mannitol, antioxidant+

| C. | DOCUMENTS CONSIDERED TO BE RELEVANT |
|---|---|

| Category* | Citation of document, with indication, where appropriate, of the relevant passages | Relevant to claim No. |
|---|---|---|
| PX | CN 116458647 A (CHINA YUANDA GROUP CO., LTD.) 21 July 2023 (2023-07-21) claims 1-10 | 1-9 |
| PX | CN 116439376 A (CHINA YUANDA GROUP CO., LTD.) 18 July 2023 (2023-07-18) description, paragraphs 27-116 | 1-9 |
| X | 周帅 等 (ZHOU, Shuai et al.). "高效液相法测定赤藓糖醇的含量 (Non-official translation: Determination of Content of Erythritol by HPLC)" 食用菌学报 (Acta Edulis Fungi), Vol. 19, No. (03), 15 September 2012 (2012-09-15), pages 83-85<br>page 83, left-hand column, paragraph 1, and page 85, right-hand column, lines 2-9 | 1-2, 4, 6-9 |

☑ Further documents are listed in the continuation of Box C. ☑ See patent family annex.

| * | Special categories of cited documents: |
|---|---|
| "A" | document defining the general state of the art which is not considered to be of particular relevance |
| "D" | document cited by the applicant in the international application |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| **06 September 2024** | **12 September 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | |
| | Telephone No. |

Form PCT/ISA/210 (second sheet) (July 2022)

## INTERNATIONAL SEARCH REPORT

| | International application No. |
|---|---|
| | **PCT/CN2024/099118** |

**C. DOCUMENTS CONSIDERED TO BE RELEVANT**

| Category* | Citation of document, with indication, where appropriate, of the relevant passages | Relevant to claim No. |
|---|---|---|
| X | 周帅 等 (ZHOU, Shuai et al.). "食用菌菌丝体中糖醇类成分含量测定的研究 (Non-official translation: Research on Determination of Alditol Content in Edible Fungal Mycelia)" 菌物学报 (Mycosystema), Vol. 32, No. (05), 15 September 2013 (2013-09-15), pages 862-867 page 863, left-hand column, last paragraph, and page 866, left-hand column, last paragraph -right-hand column, paragraph 1 | 1-2, 4, 6-9 |
| Y | 周帅 等 (ZHOU, Shuai et al.). "高效液相法测定赤藓糖醇的含量 (Non-official translation: Determination of Content of Erythritol by HPLC)" 食用菌学报 (Acta Edulis Fungi), Vol. 19, No. (03), 31 December 2012 (2012-12-31), pages 83-85 page 83, left-hand column, paragraph 1, and page 85, right-hand column, lines 2-9 | 3-9 |
| Y | CN 116200433 A (QINGHAI ZHUFENG CORDYCEPS SINENSIS MATERIAL CO., LTD.) 02 June 2023 (2023-06-02) description, paragraphs 77-103 | 3-9 |
| Y | CN 101096641 A (HANGZHOU ZHONGMEI HUADONG PHARMACEUTICAL CO., LTD.) 02 January 2008 (2008-01-02) description, page 3, paragraph 3-page 5, line 4 | 5-9 |
| Y | CN 113388567 A (QINGHAI ZHUFENG CORDYCEPS SINENSIS MATERIAL CO., LTD.) 14 September 2021 (2021-09-14) abstract, and description, paragraphs 132-138 | 5-9 |
| Y | 周帅 等 (ZHOU, Shuai et al.). "食用菌菌丝体中糖醇类成分含量测定的研究 (Non-official translation: Research on Determination of Alditol Content in Edible Fungal Mycelia)" 菌物学报 (Mycosystema), Vol. 32, No. (05), 15 September 2013 (2013-09-15), pages 862-867 page 863, left-hand column, last paragraph, and page 867, left-hand column, last paragraph -right-hand column, paragraph 1 | 3-9 |
| A | CN 108384726 A (HANGZHOU ZHONGMEI HUADONG PHARMACEUTICAL CO., LTD.) 10 August 2018 (2018-08-10) abstract | 1-9 |
| A | CN 109355204 A (MA LAN) 19 February 2019 (2019-02-19) abstract, and claims 1-2 | 1-9 |
| A | US 2010021426 A1 (WANG YACHUN et al.) 28 January 2010 (2010-01-28) claims 1-43 | 1-9 |
| A | JP 2004242506 A (OUBIKEN:KK) 02 September 2004 (2004-09-02) abstract | 1-9 |
| A | Edited by CHINESE PHARMACOPOEIA COMMISSION. "百令胶囊 (Bailing Capsule)" 中华人民共和国药典 2015年版 一部 (Pharmacopoeia of The People's Republic of China. 2015, Volume 1). 31 January 2010 (2010-01-31), pages 681-682 page 681, right-hand column, penultimate lines 5-11, and page 682, right-hand column, lines 9-10 and 18-21 | 1-9 |

Form PCT/ISA/210 (second sheet) (July 2022)

## INTERNATIONAL SEARCH REPORT
### Information on patent family members

| International application No. |
|---|
| **PCT/CN2024/099118** |

| Patent document cited in search report | | | Publication date (day/month/year) | Patent family member(s) | | | Publication date (day/month/year) |
|---|---|---|---|---|---|---|---|
| CN | 116458647 | A | 21 July 2023 | None | | | |
| CN | 116439376 | A | 18 July 2023 | None | | | |
| CN | 116200433 | A | 02 June 2023 | None | | | |
| CN | 101096641 | A | 02 January 2008 | None | | | |
| CN | 113388567 | A | 14 September 2021 | None | | | |
| CN | 108384726 | A | 10 August 2018 | None | | | |
| CN | 109355204 | A | 19 February 2019 | None | | | |
| US | 2010021426 | A1 | 28 January 2010 | US | 8722056 | B2 | 13 May 2014 |
| JP | 2004242506 | A | 02 September 2004 | None | | | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202310716796X
- WO 2014055035 A
- CN 103444434 A
- CN 103430777 A

- CN 101096641 A
- CN 108553487 A
- CN 108384726 A


**Non-patent literature cited in the description**

- **SHIN-YU CHEN et al.** Contents of lovastatin, γ-aminobutyric acid and ergothioneine in mushroom fruiting bodies and mycelia. *Food Science and Technology*, 2012, vol. 47, 274-278
- **WI YONG LEE et al.** Ergothioneine Contents in Fruiting Bodies and Their Enhancement in Mycelial Cultures by the Addition of Methionine. *Mycobiology*, 2009, vol. 37 (1), 43-47
- **BAI-XIONG et al.** Enhancement of ergothioneine production by discovering and regulating its metabolic pathway in Cordyceps militaris. *Microb Cell Fact*, 23 August 2022, vol. 21 (1), 169
- **HUI-CHEN LO et al.** A Systematic Review of the Mysterious Caterpillar Fungus Ophiocordyceps sinensis in DongChongXiaCao and Related Bioeffective components. *Journal of Traditional and Complementary Medicine*, 2013, vol. 1 (3), 16-32
- **SHIN-YI LIN et al.** Comparative Study of Contents of Several Bioactive Components in Fruiting Bodies and Mycelia of Culinary-Medicinal Mushrooms. *International Journal of Medicinal Mushrooms*, 2013, vol. 3 (15), 315-323
- **NACHSHOL COHEN et al.** Chemical Composition and Nutritional and Medicinal Value of Fruit Bodies and Submerged Cultured Mycelia of Culinary-Medicinal Higher Basidiomycetes Mushrooms. *International Journal of Medicinal Mushrooms*, 2014, vol. 3 (16), 273-291

- *Technical Specifications for Pollution-Free Cultivation and Production of Chinese Medicinal Materials*, 2018
- **QIAN JIA et al.** Study on In Vitro Antioxidant Effect of 5'-acid Adenosine (5'-AMP). *Food & Machinery*, January 2008, vol. 24 (1)
- **CHEN JIAMING et al.** Effects of Different Carbon Sources and Nitrogen Sources on Solid Fermentation Process of Hirsutella sinensis. *Edible Fungi of China*, 2017, vol. 37 (1), 55-60
- **ZHANG PING et al.** Research Progress on Quality Evaluation and Control of Cordyceps sinensis Fermentation Products. *Chinese Pharmaceutical Journal*, July 2021, vol. 56 (14)
- **LI HEYU et al.** Determination of Five Nucleoside Components in Cordyceps sinensis. Products and Quality Analysis of Cordyceps militaris. *Chinese Herbal Medicines*, 2018, vol. 22 (49), 5410-5417
- **QIAN ZHENGMING et al.** Study on Transformation Pathway of Adenosine During Water Extraction of Cordyceps sinensis. *World Journal of Traditional Chinese Medicine*, 2016, vol. 5 (11), 758-762
- **FENG YANHONG et al.** Effects of Adenosine on Isolated Heart Functions and Myocardial Cell Apoptosis in Rats. *Shandong Medicine Journal*, 2013, vol. 53 (46)